(19) Europäisches Patentamt
European Patent Office
Office européen des brevets

(11) **EP 4 421 816 A1**

(12) **EUROPEAN PATENT APPLICATION**

(43) Date of publication:
**28.08.2024 Bulletin 2024/35**

(21) Application number: **23157914.5**

(22) Date of filing: **22.02.2023**

(51) International Patent Classification (IPC):
***G16H 15/00*** *(2018.01)*

(52) Cooperative Patent Classification (CPC):
**G16H 15/00;** G16H 30/00

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Siemens Healthineers AG**
**91301 Forchheim (DE)**

(72) Inventors:
• **Farri, Oladimeji**
**Upper Saddle River, 07458 (US)**

• **Kumar Karn, Sanjeev**
**Plainsboro, 08536 (US)**
• **Ghosh, Rikhiya**
**Livingston, 07039 (US)**

(74) Representative: **Schwarz, Claudia**
**Schwarz + Kollegen**
**Patentanwälte**
**Heilmannstraße 19**
**81479 München (DE)**

Remarks:
Amended claims in accordance with Rule 137(2) EPC.

(54) **TECHNIQUE FOR SENSOR DATA BASED MEDICAL EXAMINATION REPORT GENERATION**

(57) A technique for generating a medical examination report from sensor data (316) of a medical examination is provided. Sensor data (316) from a set of sensors (310; 312) are received (S102) in relation to the medical examination (302). The sensors comprise a microphone (310). The sensor data comprise audio data (316) with utterances (308-1; 308-2) by a medical professional (304) and patient representative (306). The sensor data are processed (S104), with the audio data transformed into text (318). A verbatim report (318) of the medical examination is generating (S106). The verbatim report comprises each excerpt (322; 326) of the text assigned to the medical professional or to the patient representative. The verbatim report is converted (S108) into a summarizing medical examination report. The summary comprises vocabulary, and/or text excerpts, by accessing a predetermined ontology database (320; 324). The medical examination report is stored (S118) in an electronic medical report database.

FIG 3

**Description**

Radiation oncology conventionally involves significant documentation of patient encounters during the courses of radiotherapy. Relevant patient data need to be extracted and populated in cancer registries to aid surveillance, clinical trial recruitment and downstream epidemiological research. Documentation of patient encounters and populating cancer registries are conventionally manual, repetitive and laborious tasks that present a sizeable administrative burden for radiation oncology teams. Studies have shown that physicians in general spend twice as much time documenting compared to direct patient interaction, which has resulted in physician burnout in many cases [1, 2]. Moreover, manual documents by the physicians are prone to be incomplete, and/or at least partially incorrect.

The introduction of technology that can listen-in on the interactions during the patient encounter (e.g., voice activated smart speakers in the visit room) has allowed for automated generation of transcripts reflecting these encounters. However, the conventional verbatim transcription of the entire dialogue by such speech recognition technology do not yield comprehensible clinical notes, as verbatim transcriptions are conventionally long, verbose, topic-wise entangled and often uninterpretable [2]. Contrarily, real-world visit notes are terse and written in such a way that physicians can comprehend the content. Therefore, there is a need to contextually and constructively develop the clinical documents out of voice-automated clinical transcripts in a way that makes the contents actionable for the physician.

Furthermore, the privacy concerns associated with listening-in on physician-patient interactions cannot be understated, especially around institutional privacy concerns, i.e., about the way personal data and/or patient data is accessed and persisted by the company providing the smart speaker, third parties that may need to use and/or process the data, and the government's oversite around the data [3]. Given the recent push for "privacy by design" involving visual representations of how, and which, personal data and/or patient data will be processed [4], there is a need to follow such guidelines and ensure transparency.

Meeting summarization, a natural language processing (NLP) task, requires generating a concise and easily consumable summary out of key utterances from audio recordings, also transcripts and video, of a multi-participant meeting. Approaches for meeting summarization broadly fall under two categories: extractive (based on selection of relevant original and/or verbatim utterances) and abstractive (further compressing and/or paraphrasing the relevant original utterances for better contextual output) [5].

Several conventional solutions have been implemented to minimize the documentation and clerical burden experienced by physicians, and by extension radiation oncologists. These include the use of medical scribes and transcription services. Moreover, the more recent use of voice recognition via dictation software for documentation in the electronic health record (EHR) has not been shown to improve the time and effort invested by physician in clerical and administrative tasks [6]. However, issues with system integration and workflow adaptation have emerged as barriers to using automated speech recognition for EHR documentation while avoiding patient safety-related errors [7].

With respect to the use of meeting (or dialogue) summarization techniques in medicine, Molenaar *et al.* developed Care2Report, a pipeline for automated report generation from the interactions between general practitioners (GPs) and patients. The pipeline relies on information extraction (in the form of triples) with subsequent mapping to ontologies or clinical guidelines, and report generation is based on the normalized triples. However, the quality of the reports is significantly affected by several factors including the triple extraction errors and incompleteness of the ontology [8].

It is therefore an object of the present invention to provide a solution for, in particular automatically and/or in a timesaving manner, generating an accurate and/or complete medical examination report of a medical examination. Alternatively or in addition, it is an object of the present invention to ensure data privacy of patient data when, e.g., automatically, generating an accurate and/or complete medical examination report.

This object is solved by a method for generating a medical examination report from sensor data of a medical examination, by a computing device, by a (e.g., distributed) system, by a computer program (also denoted as computer program product) and a computer-readable medium, according to the appended independent claims. Advantageous aspects, features and embodiments are described in the dependent claims and in the following description together with advantages.

In the following, the solution according to the invention is described with respect to the claimed method as well as with respect to the claimed computing device. Features, advantages, and/or alternative embodiments herein can be assigned to the other claimed objects (e.g., the system, the computer program or a computer-readable medium), and vice versa. In other words, claims for the computing device, and/or for the (e.g., distributed) system, can be improved with features described or claimed in the context of the method, and vice versa. In this case, the functional features of the method are embodied by structural units of the computing device, and/or of the (e.g., distributed) system, and vice versa, respectively.

As to a method aspect, a (in particular computer implemented) method for generating a medical examination report from sensor data of a medical examination is provided. The method comprises a step of receiving sensor data from a set of sensors in relation to a medical examination. The set of sensors comprise at least one microphone. The sensor data comprise at least audio data (e.g., captured by the at least one microphone). The audio data comprise utterances by a medical professional and a patient representative participating in the medical examination.

The method further comprises a step of processing the received sensor data according to the type of sensors. The processing comprises at least transforming the audio data into text (e.g., initially without distinguishing between different speakers, in particular without distinguishing if an utterance stems from the medical professional or the patient representative). The method further comprises a step of generating a verbatim report of the medical examination based on the processed sensor data. The verbatim report comprises each excerpt of the text being assigned to the medical professional or the patient representative. The method further comprises a step of converting the generated verbatim report into a medical examination report. The medical examination report comprises an (e.g., abbreviated) summary of the verbatim report. The summary comprises vocabulary, and/or text excerpts, by accessing a predetermined ontology database in relation to a medical field of the medical examination (e.g., a radiological oncology database).

The method still further comprises a step of storing the medical examination report in an electronic medical report database.

Providing a medical examination report (also denoted as meeting summarization) may be explored under the unimodal (e.g., only using audio data, also denoted as verbal information) and/and multi-modal (e.g., comprising verbal and non-verbal, such as video and/or motion, information) settings. Due to the radiology oncology visit being an example of a medical examination (also denoted as meeting) between multiple participants and/or actors (e.g., patient, radiation oncologist, and/or other individuals present in the visit room, e.g., patient's relatives; briefly summarized also as patient representative and medical professional), it can be advantageous to automatically generate medical examination report (also: meeting summarization, and/or visit note), which are meaningful, accurate (also: correct), and/or complete.

By the generating and the storing of the, in particular accurate and/or complete, medical examination report using the (e.g., abbreviated) summary of the verbatim report, (e.g., all) relevant information and documentation of the medical examination can be (e.g., automatically, and/or in a timesaving manner) assembled and preserved in a comprehensive and concise form. Thereby, at minimal cost of electronic memory space, the accurate and/or complete medical examination report can be kept available for any future treatment, therapy planning, and/or consultation tailored to the patient. The electronic storage of the medical examination report further improves fast retrieval and transfer (e.g., securely, encrypted, and/or password protected), in particular across medical facilities, clinical sites, and/or medical specializations, e.g., facilitating holistic treatments. Thereby, the patient outcome can be improved long-term.

Alternatively or in addition, by the inventive technique, the conventional gap (and/or shortcomings) of an automated generation of clinical documentation, and/or medical examination reports, by using smart communication technologies, such as smart speaker (and/or comprising a smart microphone), (e.g., video) camera and motion sensors (e.g., individually or collectively as the set of sensors) is bridged (and/or addressed), and novel, in particular multi-modal, abstractive meeting summarization techniques are leveraged. Further alternatively or in addition, the inventive technique can incorporate medical professional (e.g., physician) validation of the output of the steps of processing the received sensor data, generating the verbatim report, and/or converting the generated verbatim report into the medical examination report (collectively also denoted as meeting summarization algorithm), as well as visualization of the transcribed patient representative's (e.g., the patient's) utterances towards obtaining patient representative consent (in particular in line with privacy by design principles, e.g., including deleting all instances of data once the purpose for which it is collected has been accomplished, and/or a predetermined period of time has passed).

The visualization may be provided by means of a digital twin of the patient (also denoted as digital twin app of the patient, or briefly: digital twin app), e.g., running on a patient's electronic device (briefly: patient's device).

The inventive technique is not limited to being applied to radiation oncology visits (as examples of medical examinations). Alternatively or in addition, the inventive technique can be utilized in out-patient, and/or in-hospital, visit scenarios (as examples of medical examinations).

Alternatively or in addition, the inventive technique removes the limitations of conventional triple extraction using a multi-modal end-to-end trainable system, incorporates expert-in-the-loop (e.g., medical professional, in particular physician) verification of the reports, and/or removes potential privacy concerns of patients.

The medical examination may comprise a medical consultation. Alternatively or in addition, the medical examination may comprise a multi-participant meeting.

The medical examination may take place in the medical professional's office (e.g., a doctor's office). E.g., the set of sensors may be deployed in the medical professional's office.

The medical professional may comprise a medical practitioner (also: physician, and/or medical doctor, briefly: doctor). Alternatively or in addition, the medical professional may comprise an expert healthcare professional, and/or an assisting person (e.g., a medical assistant, physician assistant, nurse, and/or dental hygienist). Further alternatively or in addition, the medical professional may comprise two or more persons, e.g., a medical practitioner and an assisting person.

The medical professional may be specialized in the field of the medical examination, e.g., as radiologist, (in particular radiological, and/or radiation) oncologist, cardiologist, neurologist, nephrologist, and/or endocrinologist.

The patient representative may comprise the patient (e.g., himself and/or herself). Alternatively or in addition, the patient representative may comprise a person authorized to act on behalf of the patient (e.g., a parent, relative, carer, and/or caretaker), in particular in case the patient is underage and/or impaired. Further alternatively or in addition, the patient representative may comprise two or more persons, e.g., the patient and one or more accompanying persons (e.g., comprising a person authorized to act on behalf of the patient).

The medical examination report may be part of, and/or may be comprised in, an electronic health record (EHR). The EHR may alternatively be denoted as electronic medical report (EMR). Alternatively or in addition, the electronic medical report database may comprise the EHR, and/or EMR.

The set of sensors may comprise the at least one microphone and at least one further sensor. The at least one further sensor may comprise a (e.g., video) camera, and/or a motion sensor. Alternatively or in addition, the microphone may be integrated with the at least one further sensor, e.g., with the (in particular video) camera. Further alternatively or in addition, the microphone may be comprised in a (e.g., voice activated, and/or smart) speaker.

Performing the method using two or more sensors may be denoted as multi-modal. E.g., the (in particular multi-modal) set of sensors may comprise a microphone and a (e.g., video) camera.

The sensor data may also be denoted as, in particular digital, recording.

Processing the audio data received from the microphone may comprise performing speech recognition, in particular by means of a speech recognition program (e.g., without identifying the speaker, in particular without identifying which parts of the audio data originate from the medical professional and which parts of the audio data originate from the patient representative). Alternatively or in addition, the processing of the audio data received from the microphone may comprise identifying any one of the participants of the medical examination (e.g., the medical professional, and/or the patient representative), e.g., by means of a speech pattern, and/or a frequency pattern, of the participant.

Alternatively or in addition, processing the received sensor data of the (e.g., video) camera (which may also be denoted as visual data) may comprise facial recognition, recognition of motions (also: movements), in particular of the lips, recognition of gestures, and/or any further identification of any one of the participants of the medical examination (e.g., the medical professional, and/or the patient representative). The further identification may comprise, e.g., identifying the medical professional according to an attributed position (e.g., a side of a desk in a doctor's office), and/or according to professional clothing. Alternatively or in addition, the further identification may comprise, e.g., identifying the patient representative according to an attributed position (e.g., another side of a desk in a doctor's office, and/or an examination chair), and/or according to non-professional clothing (e.g., not wearing clothes resembling a medical work coat in shape and/or color).

Further alternatively or in addition, processing the received sensor data of the motion sensor (which may comprise visual data) may comprise recognition of gestures of any participant of the medical examination (e.g., the medical professional, and/or the patient representative). Alternatively or in addition, the processing of the motion sensor may comprise identifying any one of the participants of the medical examination (e.g., the medical professional, and/or the patient representative), e.g., according to their attributed position, and/or according to characteristic gestures (in particular performed by the medical professional).

The verbatim report may also be denoted as (e.g., verbal, verbatim, word-for-word, and/or literal) transcription and/or transcript.

Generating the verbatim report may comprise combining the received sensor data from the microphone, and/or from two or more microphones, and/or from the one or more further sensors. E.g., the generating of the verbatim report may comprise complementing (and/or combining) the audio data with visual data. The combining of the audio data with the visual data may comprise assigning each utterance, and/or text excerpt, to the medical professional, and/or to the patient representative.

The utterances may also be denoted as statements, pronouncements, expressions, and/or comments.

Converting the generated verbatim report into the medical examination report may comprise applying natural language processing (NLP) to the verbatim report. Alternatively or in addition, converting the generated verbatim report into the medical examination report may be performed by means of an artificial intelligence (AI), neural network (NN), deep learning (DL), and/or reinforcement learning (RL).

The summary of the verbatim report may also be denoted as abstractive (and/or abbreviated) summary.

The (e.g., abstractive, and/or abbreviated) summary may be generated (e.g., separately, and/or independently) for the excerpts of the text assigned to the medical professional and to the patient representative. E.g., the conversion of the verbatim report may preserve the utterances by the patient representative word-by-word, and/or may convert (in particular abbreviate, and/or transform into, especially standardized, expert terminology) the utterances by the medical professional, in particular using NLP.

The predetermined ontology database may comprise a database of medical expressions and/or of expert terminology (e.g., words, multiword expressions, and/or phrases). Alternatively or in addition, the predetermined ontology database may comprise (e.g., only, and/or may be reduced to) the medical expressions, and/or expert terminology, assigned to the medical specialization (e.g., radiology, and/or, in particular radiological, oncology) of the medical professional.

Storing the medical examination report in an electronic medical report database may comprise storing the medical examination report (e.g., centrally) at a medical facility (also: clinical site, clinic, hospital, and/or doctor's office). Alternatively or in addition, storing the medical examination report in an electronic medical report database may comprise storing the medical examination report (e.g., locally) in a digital twin of the patient, and/or on a patient's device.

The set of sensors may comprise at least one further sensor, e.g., in addition to a microphone. The at least one further sensor may comprise a camera, in particular a video camera. Alternatively or in addition, the at least one further sensor may comprise a motion sensor.

The method may further comprise a step of providing the text excerpts of the converted medical examination report, which are assigned to the patient representative, to the patient representative for approval. The method may still further comprise a step of receiving a user input by the patient representative. The user input may be indicative of approval, and/or rejection, in particular of the correctness, of the provided text excerpts assigned to the patient representative.

Any user input (e.g., by the patient representative, and/or by the medical professional) may be received via a (e.g., graphical) user interface (UI, in particular GUI).

The received user input (e.g., by the patient representative) may comprise an approval of a subset, and/or of the full set, of text excerpts assigned to the patient representative. Alternatively or in addition, the received user input may comprise a rejection of a subset, and/or of the full set, of text excerpts assigned to the patient representative. The rejection may be indicative of the patient representative not consenting to digitally storing the corresponding text excerpts. Alternatively or in addition, the rejection may be indicative of the patient representative assessing the corresponding text excerpts as incorrectly transformed into text (also: transcribed).

Alternatively or in addition, the user input (e.g., by the patient representative, and/or by the medical professional) may comprise corrections to the provided text excerpts. E.g., the UI (and/or GUI) may comprise a (in particular text) editor for modifying the provided text excerpts, and/or for adding comments. Alternatively or in addition, comments may be selected based on predefined icons, and/or a predefined scale (e.g., indicating the correctness of the transcript).

The receiving of the user input by the patient representative may be time restricted. E.g., after a time period (also: lapse of time), e.g., two days, without user input responsive to the provided text excerpts, the provided text excerpts may be classified as approved.

Alternatively or in addition, the method may further comprise a step of providing the text excerpts of the converted medical examination report, which are assigned to the medical professional, to the medical professional for verification. The method may still further comprise a step of receiving a user input, by the medical professional, indicative of a validation of the provided text excerpts assigned to the medical professional.

The providing of the text excerpts of the converted medical examination report to the medical professional may comprise providing the full converted medical examination report (in particular also comprising the text excerpts assigned to the patient representative) to the medical professional. The providing of the full converted medical examination report may be subject to the approval of the patient representative (e.g., by the step of receiving the user input by the patient representative).

The user input by the medical professional may comprise corrections, additions, and/or one or more (e.g., manual) annotations.

The method may further comprise a step of temporarily storing intermediate data in a private cloud. The intermediate data may comprise the received sensor data, the generated verbatim report and/or at least parts of the converted medical examination report. The method may still further comprise a step of deleting the temporarily stored intermediate data from the private cloud after a predetermined period of time, after a rejection by the patient representative, and/or after a verification by the medical professional.

The private cloud may also be denoted as private access cloud (PAC), and/or as protected cloud. The protection may comprise a password, an encryption, and/or a firewall. Alternatively or in addition, the private cloud may serve for privacy protection of (e.g., sensitive) patient data.

The temporarily storing may also be denoted as intermediate storing.

Storing the medical examination report in an electronic medical report database (e.g., in the long term) may comprise storing the medical examination report in a centralized memory of a medical facility, in particular of the medical facility where the medical examination was performed.

Alternatively of in addition, a copy of the medical examination report may be sent (e.g., securely) from the (e.g., specialized) medical professional to a general practitioner (also: family doctor) and stored in a (e.g., centralized) memory there. Further alternatively or in addition, the medical examination report may be sent, e.g., upon request, to another medical professional of the same, and/or a different, medical field of the medical examination.

The method may further comprise a step of outputting the stored medical examination report.

Outputting the stored medical examination report may comprise retrieving the medical examination report from the electronic medical report database, e.g., for display and/or information at a later consultation.

Alternatively or in addition, storing the medical examination report in an electronic medical report database may comprise storing the medical examination report in a digital twin of the patient. Alternatively or in addition, the providing of the text excerpts assigned to the patient representative and the receiving of the user input by the patient representative may be performed using the digital twin of the patient.

The digital twin of the patient may comprise an interface, memory, and/or database, for storing, and/or viewing by the patient representative, health-related metrics, and/or documents of the patient. Alternatively or in addition, the digital twin may comprise, and/or store, a patient health record. The patient health record may comprise the patient's daily activities (e.g., sleep, exercise, and/or diet), the patient's medical, and/or surgical, history, the patient's vital signs (e.g., received from a wearable device), the patient's laboratory (short: lab) test results, and/or the patient's medical imaging data (briefly: medical images).

Alternatively or in addition, the digital twin may comprise the text excerpts of the converted medical examination report, and/or of the verbatim report of the medical examination, assigned to the patient representative. Approving (also: consenting) or rejecting (also: refusing) the text excerpts assigned to the patient representative may be performed by means of the digital twin.

The text excerpts of the converted medical examination report assigned to the patient representative may only be forwarded to, and/or provided to, the medical professional upon approval (also: consent) of the patient representative.

The assignment of each excerpt of the text to the medical professional or the patient representative in the step of generating a verbatim report may be based on filtering of the audio data according to a frequency pattern, and/or according to a low pass filter, and/or a high pass filter. The filtered audio data may be classified according to characteristics determined by the filtering. The classifying characteristics may be assigned to the medical professional and/or the patient representative.

Alternatively or in addition, the assignment of each excerpt of the text to the medical professional or the patient representative in the step of generating a verbatim report may be based on extracting time stamps from the audio data and from the further sensor data, combining the audio data and the further sensor data, in particular visual data, according to the extracted time stamps, and assigning each excerpt of the text to the medical professional or the patient representative according to the combination of the audio data and the further sensor data, in particular the visual data, per time stamp.

The method may further comprise a step of identifying, based on the processed sensor data, medical imaging data that were examined during the medical examination. Alternatively or in addition, the method may further comprise a step of appending the medical imaging data to the medical examination report.

The medical imaging data may be obtained (e.g., prior to the medical examination) by means of medical imaging. Alternatively or in addition, the medical imaging data may be obtained in relation to the patient, who is the subject of the medical examination.

The medical imaging may comprise a computed tomography (CT) scan, a magnetic resonance imaging (MRI) scan, an ultrasound (US) scan, a positron emission tomography (PET) scan, a single photon emission computed tomography (SPECT) scan, and/or an X-ray scan (also denoted as radiography scan). Alternatively or in addition, the medical imaging data may be stored in a picture archiving and communication system (PACS), e.g., at the medical facility, clinical site, and/or location, of the medical professional's office.

Examining the medical imaging data may comprise viewing, and/or discussing, the medical imaging data, in particular during the medical examination.

The medical imaging data may be appended to, and/or included in, the medical examination report. Alternatively or in addition, appending the medical imaging data to the medical examination report may comprise including a link to a storage location of the medical imaging data, e.g., within a medical facility and/or clinical site.

Alternatively or in addition, the medical professional may correct the identification of, and/or may add, medical imaging data to be appended to the medical examination report.

Converting the generated verbatim report into a medical examination report may be based on a trained artificial intelligence (AI), a trained neural network (NN), deep learning (DL), and/or reinforcement learning (RL).

The NN may comprise a bidirectional autoregressive transformer (BART) and/or at least one decoder, preferably one decoder per sensor.

Alternatively or in addition, the AI, NN, DL, and/or RL may be based on NLP, entity recognition (in particular of medical terminology), and/or entity linking (in particular of medical terminology) according to the ontology database. Further alternatively or in addition, the AI, NN, DL, and/or RL may be based on mapping sequences (also denoted as multiword expressions, and/or phrases) to medical concepts, and/or medical terminology comprised in the ontology database.

The training of the AI and/or the NN, and/or the learning of the DL and/or the RL, may be based on training data sets. Each training data set may comprise sensor data and an associated, in particular at least manually compiled, medical examination report.

The (e.g., at least manually compiled) medical examination report within the training data set may be denoted as ground truth. The ground truth may be provided by a medical professional.

The training, and/or learning, may be based on optimizing a loss function.

The loss function may comprise a reconstruction loss. Optimizing the loss function may comprise minimizing the reconstruction loss.

Alternatively or in addition, the loss function may comprise a reward. Optimizing the loss function may comprise maximizing the reward. Alternatively or in addition, the reward may be based on an n-gram overlap of the output, by the AI, NN, DL, and/or RL, of a medical examination report with the ground truth (e.g., the at least manually compiled medical examination report).

The training, and/or learning, may be unsupervised, and/or supervised.

The medical examination may comprise a radiology, and/or an, in particular radiological, oncology examination.

As to a device aspect, a computing device for generating a medical examination report from sensor data of a medical examination is provided. The computing device comprises a sensor interface configured to receive sensor data from a set of sensors in relation to a medical examination. The set of sensors comprises at least one microphone. The sensor data comprise at least audio data (e.g., captured by the at least one microphone). The audio data comprise utterances by a medical professional and a patient representative participating in the medical examination.

The computing device further comprises a processing module configured to process the received sensor data according to the type of sensors. The processing comprises at least transforming the audio data into text. The computing device further comprises a generating module configured to generate a verbatim report of the medical examination based on the processed sensor data. The verbatim report comprises each excerpt of the text being assigned to the medical professional or the patient representative. The computing device further comprises a converting module configured to converting the generated verbatim report into a medical examination report. The medical examination report comprises a summary of the verbatim report. The summary comprises vocabulary, and/or text excerpts, by accessing a predetermined ontology database in relation to a medical field of the medical examination. The computing device still further comprises a storage interface configured to forward the medical examination report to an electronic medical report database for storing.

Any of the above modules of the computing device may also be denoted as unit. Alternatively or in addition, any combination of the processing module, the generating module, and/or the converting module may be comprised in a (in particular centralized) processing unit (e.g., a CPU). The processing unit may also briefly be denoted as processor.

As to a system aspect, a (e.g., distributed) system for generating a medical examination report from sensor data of a medical examination is provided. The (e.g., distributed) system comprises a set of sensors comprising at least one microphone. The set of sensors is configured to capture sensor data relating to a medical professional and a patient representative participating in the medical examination. The sensor data comprise at least audio data (e.g., captured by the at least one microphone). The audio data comprise utterances by the medical professional and the patient representative.

The (e.g., distributed) system further comprises a processing module configured to process the sensor data according to the type of sensors. The processing comprises at least transforming the audio data into text. The (e.g., distributed) system further comprises a generating module configured to generate a verbatim report of the medical examination based on the processed sensor data. The verbatim report comprises each excerpt of the text being assigned to the medical professional or the patient representative. The (e.g., distributed) system further comprises a converting module configured to convert the generated verbatim report into a medical examination report. The medical examination report comprises a summary of the verbatim report. The summary comprises vocabulary, and/or text excerpts. The (e.g., distributed) system further comprises an ontology database in relation to a medical field of the medical examination. The ontology database comprises the vocabulary, and/or the text excerpts for the summary of the verbatim report. The ontology database is configured to be accessed for converting the generated verbatim report into the medical examination report. The (e.g., distributed) system still further comprises a storage module configured to store the medical examination report in an electronic medical report database.

Any of the above modules of the (e.g., distributed) system may also be denoted as unit. Alternatively or in addition, any combination of the processing module, the generating module, and/or the converting module may be comprised in a (in particular centralized) processing unit (e.g., a CPU). The processing unit may also briefly be denoted as processor.

The (e.g., distributed) system may at least partially be embodied by a computing cloud (briefly: cloud). Alternatively or in addition, the processing module, the generating module, the converting module, the ontology database, and/or the electronic medical report database (and/or the storage module configured to store the medical examination report in an electronic medical report database) may be part of a computing cloud.

The (e.g., computing) cloud may be a private, and/or protected, cloud. Alternatively or in addition, the protection of the (e.g., computing) cloud (briefly: cloud protection) may comprise a password protection, firewall, and/or an access restriction (e.g., access reserved to verified devices, and/or to verified users).

By the (e.g., computing) cloud being private, and/or protected, sensible data (in particular related to the patient) may be protected.

The system may be distributed in the sense that the hardware, on which the modules and/or the interfaces are implemented, may be distributed, and/or may comprise two or more physical entities.

As to a further aspect, a computer program comprising program elements which, when the program elements are loaded into a memory of a computing device (and/or a, e.g., distributed, system), induce the computing device (and/or the, e.g., distributed, system) to carry out the steps of the method for generating a medical examination report from sensor data of a medical examination according to the method aspect is provided.

As to a still further aspect, a computer-readable medium, on which program elements are stored that can be read and executed by a computing device (and/or a, e.g., distributed, system), in order to perform steps of the method, when the program elements are executed by the computing device (and/or by the, e.g., distributed, system), according to the method aspect for generating a medical examination report from sensor data of a medical examination is provided.

The properties, features and advantages of this invention described above, as well as the manner they are achieved, become clearer and more understandable in the light of the following description and embodiments, which will be described in more detail in the context of the drawings. This following description does not limit the invention on the contained embodiments. Same components or parts can be labelled with the same reference signs in different figures. In general, the figures are not for scale.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

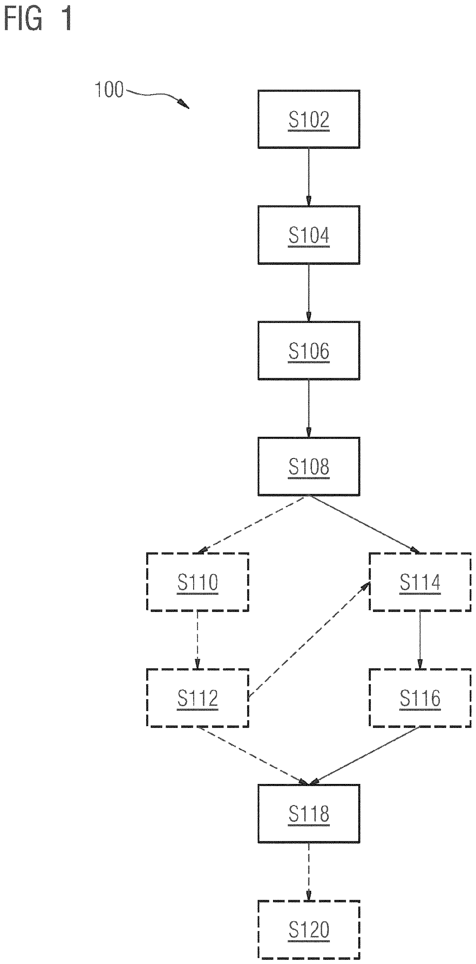

Fig. 1    is a flow chart of a method for generating a medical examination report from sensor data of a medical examination according to a preferred embodiment of the present invention;

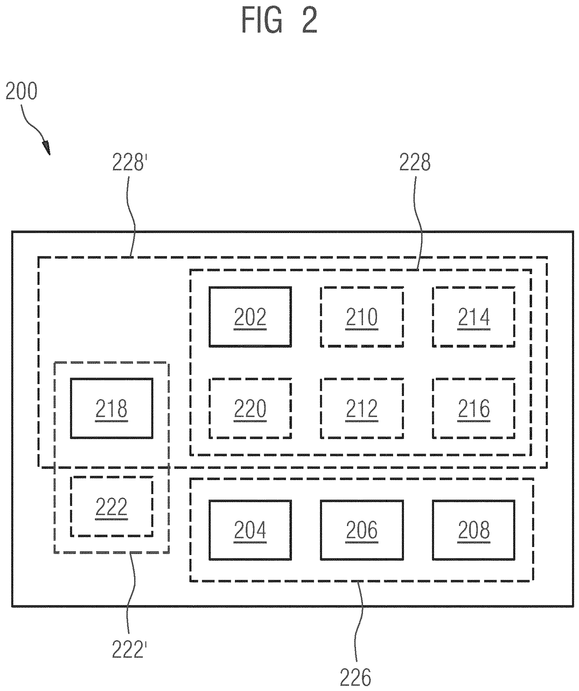

Fig. 2    is an overview of the structure and architecture of a computing device for generating a medical examination report from sensor data of a medical examination according to a preferred embodiment of the present invention, wherein the computing device may be configured to perform the method of Fig. 1;

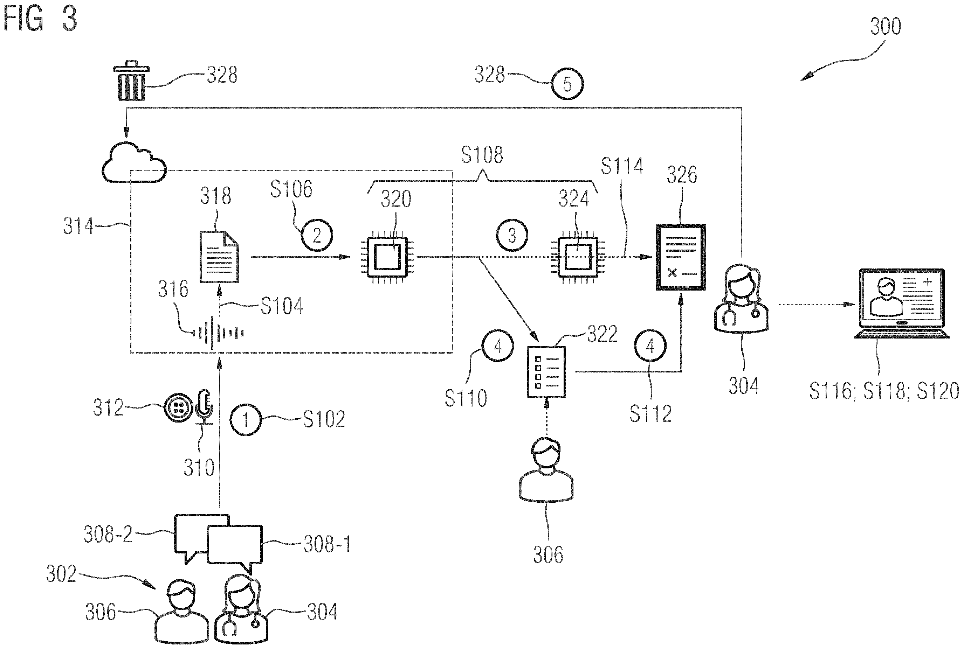

Fig. 3    is an overview of the structure and architecture of a, in particular distributed, system for generating a medical examination report from sensor data of a medical examination according to another preferred embodiment of the present invention, in which method steps, e.g., according to the method of Fig. 1 are shown as well;

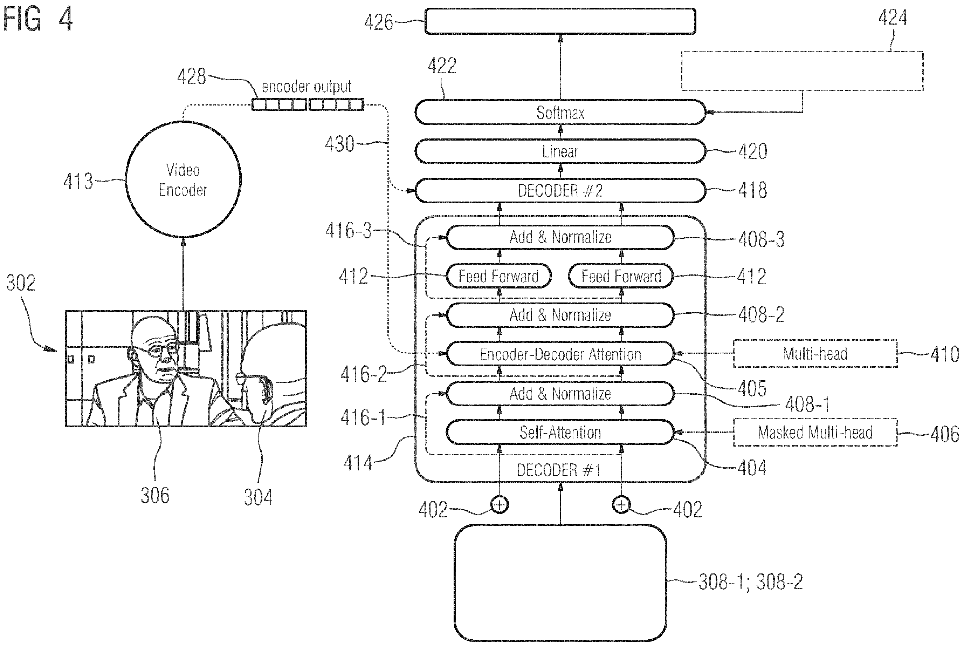

Fig. 4    is a schematic overview of a neural network architecture for processing (e.g., individually per sensor) sensor data, generating a verbatim report (e.g., collectively based on the sensor data from all sensors), and converting the verbatim report into the medical examination report, in particular according to the method of Fig. 1, wherein the neural network architecture may be comprised in the computing device of Fig. 2 and/or the, in particular distributed, system of Fig. 3; and

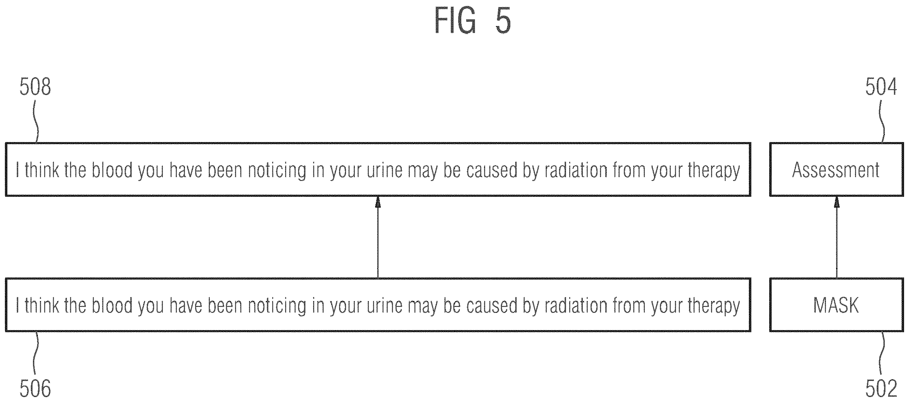

Fig. 5    is an exemplary illustration of a subjective-objective-assessment-plan (SOAP) section and/or subsection masking, which may be used when performing the method of Fig. 1.

Any reference signs in the claims should not be construed as limiting the scope.

Fig. 1 schematically illustrates a computer implemented method for generating a medical examination report from sensor data of a medical examination. The method is generally denoted by the reference sign 100.

The method 100 comprises a step S102 of receiving sensor data from a set of sensors in relation to a medical examination. The set of sensors comprises at least one microphone. The sensor data comprise at least audio data (in particular captured by the at least one microphone). The audio data comprise utterances by a medical professional and a patient representative participating in the medical examination.

The method 100 further comprises a step S104 of processing the received S102 sensor data according to the type of sensors. The processing S104 comprises at least transforming the audio data into text.

The method 100 further comprises a step S106 of generating a verbatim report of the medical examination based on the processed S104 sensor data. The verbatim report comprises each excerpt of the text being assigned to the medical professional or the patient representative.

The method 100 further comprises a step S108 of converting the generated S106 verbatim report into a medical examination report. The medical examination report comprises an (e.g., abbreviated) summary of the verbatim report.

The summary comprises vocabulary, and/or text excerpts, obtained (and/or received, and/or retrieved) by accessing a predetermined ontology database in relation to a medical field of the medical examination.

The method 100 still further comprises a step S118 of storing the medical examination report in an electronic medical report database.

Optionally, the method 100 comprises a step S110 of providing the text excerpts of the converted S108 medical examination report, which are assigned to the patient representative, to the patient representative for approval. The method 100 may further comprise a step S112 of receiving a user input by the patient representative. The user input may be indicative of approval, and/or rejection, in particular of the correctness, of the provided S110 text excerpts assigned to the patient representative.

Further optionally, the method 100 comprises a step S114 of providing the text excerpts of the converted S108 medical examination report, which are assigned to the medical professional, to the medical professional for verification. The method 100 may further comprise a step S116 of receiving a user input, by the medical professional, indicative of a validation of the provided S114 text excerpts assigned to the medical professional.

Still further optionally, the method 100 comprises a step S120 of outputting the stored S118 medical examination report.

Fig. 2 schematically illustrates a computing device for generating a medical examination report from sensor data of a medical examination. The computing device is generally referred to by the reference sign 200.

The computing device 200 comprises a sensor interface 202 configured to receive sensor data from a set of sensors in relation to a medical examination. The set of sensors comprises at least one microphone. The sensor data comprise at least audio data (e.g., captured by the at least one microphone). The audio data comprise utterances by a medical professional and a patient representative participating in the medical examination.

The computing device 200 further comprises a processing module 204 configured to process the received sensor data according to the type of sensors. The processing comprises at least transforming the audio data into text.

The computing device 200 further comprises a generating module 206 configured to generate a verbatim report of the medical examination based on the processed sensor data. The verbatim report comprises each excerpt of the text being assigned to the medical professional or the patient representative.

The computing device 200 further comprises a converting module 208 configured to convert the generated verbatim report into a medical examination report. The medical examination report comprises a summary of the verbatim report. The summary comprises vocabulary, and/or text excerpts, by accessing a predetermined ontology database in relation to a medical field of the medical examination.

The computing device 200 still further comprises a storage interface 218 configured to forward the medical examination report to an electronic medical report database for storing.

Optionally, the computing device 200 comprises a first text excerpts sending interface configured for providing the text excerpts of the converted medical examination report, which are assigned to the patient representative, to the patient representative for approval. Alternatively or in addition, the computing device 200 may comprise a first user input receiving interface 212 for receiving a user input by the patient representative. The user input may be indicative of approval, and/or rejection, in particular of the correctness, of the provided text excerpts assigned to the patient representative.

Further optionally, the computing device 200 comprises a second text excerpts sending interface 214 configured for providing the text excerpts of the converted medical examination report, which are assigned to the medical professional, to the medical professional for verification. Alternatively or in addition, the computing device 200 may comprise a second user input receiving interface 216 for receiving a user input, by the medical professional, indicative of a validation of the provided text excerpts assigned to the medical professional.

Still further optionally, the computing device 200 comprises an output interface 220 for outputting the stored medical examination report.

Any of the above modules 204; 206; 208 may also be denoted as unit.

Alternatively or in addition, any (e.g., pairwise, and/or overall) combination of the processing module 204, the generating module 206, and/or the converting module 208 may be comprised in a (in particular centralized) processing unit 226 (e.g., a CPU). The processing unit 226 may also briefly be denoted as processor 226.

Further alternatively or in addition, any one of the interfaces 202; 210; 212; 214; 216; 218 may be comprised in an external interface 228'. Alternatively or in addition, the external interface 228 may, e.g., comprise the interfaces 202; 210; 212; 214; 216.

Still further alternatively or in addition, the storage interface 218 may comprise an internal interface to a (e.g., internal) memory 222 comprising the electronic medical report database. The internal storage interface 218 and memory 222 may be collectively denoted as (e.g., internal) memory 222'.

Alternatively or in addition, the first text excerpts sending interface 210 and the first receiving user input interface 212 may be comprised in a patient representative interface.

Alternatively or in addition, the second text excerpts sending interface 214 and the second receiving user input interface 216 may be comprised in a medical professional interface.

Further alternatively or in addition, the first text excerpts sending interface 210, the first receiving user input interface 212, the second text excerpts sending interface 214 and the second receiving user input interface 216 may be comprised in a user interface.

The computing device 200 may be configured to perform the method 100.

Alternatively or in addition, a (e.g., distributed) system may comprise any one of the modules 204; 206; 208, interfaces 202; 210; 212; 214; 216; 218; 220; 228; 228', memory 222; 222', and/or processing unit 226 disclosed in the context of the computing device 200. The (e.g., distributed) system may generally be denoted by reference sign 300.

Further alternatively or in addition, the (e.g., distributed) system 300 may be cloud-based, and/or may comprise a (e.g., private) cloud.

The (e.g., distributed) system 300 may be configured to perform the method 100.

The inventive technique (e.g., comprising the method 100, computing device 200, and/or system 300) focuses on addressing the gap of automated generation of clinical documentation (in particular medical examination reports) by using smart communication technologies, such as smart speaker (and/or microphone), camera and motion sensors (independently or collectively as the set of sensors), and novel (in particular multi-modal) abstractive meeting summarization techniques. Alternatively or in addition, the inventive technique incorporates medical professional (e.g., physician) validation of the output of a meeting summarization algorithm (e.g., comprising the steps of processing S104 the received S102 sensor data, of generating S106 a verbatim report of the medical examination based on the processed S104 sensor data, and/or of converting S108 the generated S106 verbatim report into the medical examination report). Further alternatively or in addition, the inventive technique can provide a visualization of the transcribed patient representative's (e.g., the patient's) utterances towards obtaining patient representative consent (in particular in line with privacy by design principles).

The inventive technique can be applied to radiation oncology visits (as examples of medical examinations). Alternatively or in addition, the inventive technique can be utilized in out-patient, and/or in-hospital, visit scenarios (as examples of medical examinations).

The inventive technique can remove limitations of conventional triple extraction using a (in particular multi-modal) end-to-end trainable system (e.g., comprising an AI, NN, DL, and/or RL). Alternatively or in addition, the inventive technique can incorporate expert-in-the-loop (e.g., medical professional, in particular physician) verification of the medical examination reports, and/or of the clinical documentation. Alternatively or in addition, the inventive technique can disperse, extinguish, and/or obviate potential privacy concerns of patient representatives (e.g., patients).

Fig. 3 shows an exemplary embodiment of the (e.g., distributed) system 300. The system 300 in the example of Fig. 3 may alternatively be denoted as (e.g., radiation oncology) automated documentation system 300.

As shown in Fig. 3, an embodiment of the inventive technique involves using smart communication technologies (e.g., a smart speaker and/or microphone 310, and/or a further sensor 312, in particular a video camera 312, shortly also denoted as camera 312) during a medical examination 302 (also denoted as follow-up patient visit), e.g., for radiation oncology and/or radiotherapy.

The dialogue between the radiation oncologist (as embodiment of the medical professional 304) and patient (and/or including relatives of the patient present, as patient representative 302) comprising utterances 308-1 by the medical professional 304 and utterances 308-2 by the patient representative 306 are captured in the step S102 (e.g., by the microphone 310 as audio files schematically depicted at reference sign 316 in Fig. 3, and potentially as non-verbal data captured by one or more cameras 312 and/or, in particular motion, sensors 312) and sent to a cloud 314 storage.

In the particular embodiment of Fig. 3, no local (e.g., in the doctor's office) copy of the files (in particular comprising the sensor data 316) is persisted. The audio file 316 is translated S104 to a verbatim report (also: text file) 318 using a speech-to-text application programming interface (API) provided, (and/or offered) by the cloud 314 platform provider (e.g., comprising Microsoft azure cognitive services). The verbatim report (also: text file) 318 (and optionally, in particular a transcript and/or evaluation S106 of, the non-verbal data) are then sent as an input into a cloud-based deployment of a multi-modal meeting summarization model 320; 324.

In one embodiment, a state-of-the-art approach for multi-modal meeting (as the medical examination 302 with multiple sensors 310; 312 recording sensor data 316) summarization 320 is used, with an additional task of standardization 324 (e.g., corresponding to, and/or comprising, the step S108 of converting the verbatim report into the medical examination report), e.g., denoted as *summarize-normalize* paradigm. The multi-modal summarization model 320 (e.g., comprising, and/or correspond to, the method steps of processing S104 the sensor data, generating S106 the verbatim report, and/or converting the verbatim report 318 into the medical examination report) according to an embodiment is trained on millions of video frames and associated captions (also denoted as text excerpts and/or annotations, and/or comprising verbatim reports 318 and/or medical examination reports associated with the video frames) that include, which speaker (e.g., the medical professional 304 and/or the patient representative 302) said what 308-1; 308.

In an alternative embodiment, the step S108 of converting the verbatim report into the medical examination report (and/or the additional task of standardization 324) may affect, and/or concern, both the verbatim report of the utterances 308-1 of the medical professional 304 and the utterances 308-2 of the patient representative 308-2.

Fig. 4 exemplarily illustrates a neural network (NN) architecture for processing S104 the received S102 sensor data, generating S106 the verbatim report, and/or converting S108 the generated S106 verbatim report into the medical examination report. The NN architecture of Fig. 4 may also be denoted as multi-modal query-focused summarization architecture.

As exemplarily illustrated in Fig. 4, the video frames of the medical examination 302 are passed as input into a video encoder 413 that represents the pixels with vectors signifying the objects and persons in the clips. The encoded output 428 is passed 430 to various layers 405 of a decoder 414; 418 that include a vector representation of the associated caption for each video clip (e.g., comprising the video frames).

Alternatively or in addition, an input embedding 402 may refer to a vector representation of a token, e.g., word embeddings.

At reference sign 402, positional embeddings, e.g., a vector representation of the absolute and/or relative position of a token in a sequence of tokens, is input into the first decoder 414 and/or the second decoder 418. The positional embeddings 402 are combined with the input embeddings to the first decoder 414 and/or the second decoder 418, e.g., vector representation of the text-based utterances 308-1; 308-2.

Herein, a token may comprise a word, and/or a sequence of (in particular consecutive) words, of sensor data captured by a microphone 310. Alternatively or in addition, one or more (e.g., collection and/or accumulation of) pixels may comprise a gesture, and/or lip movements, captured by a further sensor, in particular a camera 312, and/or a motion sensor 312.

The positional embeddings may, e.g., correspond to, and/or may comprise, time stamps in relation to utterances 308-1; 308-2, gestures, and/or lip movement.

The self-attention sublayer 404 in the decoder 414 (and/or of the second decoder 418) of the embodiment of the NN architecture in Fig. 4 takes as input 402 a sequence of tokens (and/or representing captions, wherein a caption may refer to, and/mor may comprise, a text excerpt), $h_1,..,h_n$ (also denoted as, in particular sequence, of vectors, and/or as vector representation). Each vector is linearly transformed into the query, key and value vectors denoted by the symbols $q_i$, $k_i$ and $v_i$, respectively. All the vectors $q_i$, $k_i$ and $v_i$ are packed into the Q, K and V matrices, respectively. The matrices Q and K are initialized with random weights, $W_Q$ and $W_K$, respectively. A SoftMax function (also denoted as SoftArgMax, and/or normalized exponential function, e.g., functioning in order to convert a matrix into a probability distribution, in particular of possible outcomes) is applied to the product of Q and (in particular the transposed of) K (e.g., scaled using the square root of the dimension of K) to calculate the weight for V, i.e., $W_V$. The attention matrix for the sublayer 404 (Attention (Q, K, V)) is the summation of $V(W_V)$ from all possible combination pairs of vectors from Q and K, as displayed in Eq. (1):

$$Attention(Q, K, V) = softmax\left(\frac{Q \cdot K^T}{\sqrt{d_k}}\right)V \qquad (1)$$

In the architecture of Fig. 4, the 'add' sublayer 408-1; 408-2; 408-3 represents a residual connection between the self-attention sublayers 404 across different blocks (408-1), and between the self-attention sublayer 404 and the simple position-wise fully connected feed forward network 412 within a block. These connections force the input of the subsequent sublayer 405; 412; 418 to include the original token vectors ($h_1,..,h_n$) alongside the output of the previous sublayer 408-1; 408-2; 408-3 (e.g., decoded pixels, in particular of a camera 312 encoding 413, and tokens). The 'normalize' aspect (and/or performing the step S108 of converting the verbatim report 318 into the medical examination report) may represent a function to transform all outputs from the self-attention layers 404 into a specific dimension (e.g., $d_{model} = 512$).

The decoder 414 and/or the second decoder 418) in the NN architecture embodiment of Fig. 4 also includes the encoder-decoder attention sublayer 405. The sublayer 405 essentially functions the same way the self-attention sublayer 404 does, except that the query matrix Q is derived from the input vectors of the decoder 414; 418 (e.g., tokens from captions), while the key and value matrices (K and V, respectively) are derived from the encoder output 428 (e.g., pixels from videos, e.g., captured by a camera 312). In other words, the sublayer 405 allows the tokens to 'attend' to the pixels, unlike the self-attention sublayer 404, in which the tokens 'attend' only to themselves. In turn, there is an additional add and normalize sublayer 408-2; 408-3 which represents a residual connection between the self-attention 404 and the encoder-decoder attention sublayers 405.

The input (and/or the input embedding 402), and/or any one of the add and normalize sublayers 408-1; 408-2; 408-3 may be connected, e.g., directly, by (e.g., skip and/or shortcut) connections 416-1; 416-2; 416-3. E.g., in addition to receiving the output from the self-attention sublayer 404, the add and normalize sublayer 408-1 may, in particular directly, receive the input embedding 402 through the connection 416-1. Alternatively or in addition, a later add and normalize sublayer 408-2; 408-3 may, in addition to the output of the directly preceding sublayer 405; 412, receive the output from the preceding add and normalize sublayer 408-1; 408-2 through the corresponding connection 416-2; 416-3.

The self-attention sublayer 404 may comprise, and/or may be supplied by, a masked multi-head 406. Alternatively or in addition, the encoder-decoder attention sublayer 405 may comprise, and/or may be supplied by, a multi-head 406.

The final output from the decoder (e.g., the first decoder 414, and/or the second decoder 418) in the NN architecture embodiment of Fig. 4 is pushed to a SoftMax layer 422, via a linear sublayer 420. The SoftMax layer 422 predicts a response 426 to an input query 424 by retrieving a span of tokens (that in particular comprise the answer), the input video (e.g., captured by the camera 312 of Fig. 3) and captions 308-1; 308-2 (e.g., captured by the microphone 310 of Fig. 3). In this way a, summary 426 (and/or medical examination report) of a conversation 308-1; 308-2 from the input video (in particular including audio data 316) can be provided.

The second decoder 418 in the NN architecture embodiment of Fig. 4 may be an, e.g., exact, replica of the first decoder 414 shown in greater detail in Fig. 4.

The resulting model (e.g., comprising, and/or corresponding to the trained, and/or learned, NN, AI, DL, and/or RL) for query-focused summarization 426 (and/or medical examination report generation), when streamlined to the exemplary use case of a medical examination report (in particular a radiation oncology documentation), focuses on generating a concise representation of a document (e.g., the verbatim report 318) based on a specific input query [9]. E.g., *'assessment'* may denote the query needed to generate the medical examination report, in particular the summary of the radiation oncologist's evaluation and/or diagnosis.

It is noted that [9] only focuses on extracting and/or retrieving Wikipedia sentences to comprise a summary based on a query, whereas the inventive technique involves abstracting and/or generating a medical examination report (e.g., using concise, and/or standard, report language of the medical field of the medical examination) based on a query.

The *summarize-normalize* paradigm (e.g., embodied by the converting module 208, and/or assigned for performing the step S108 of converting the generated S106 verbatim report into the medical examination report) according to an embodiment comprises generating an abstractive summary 326 (e.g., denoted as *'summarize',* and/or as summary of the medical professional's text excerpts) from a medical professional 304 (e.g., a radiation oncologist's) utterances 308-1 related to the query, and then summarizing all other utterances 308-2 as the *patient utterance summary* 322 (also denoted as the patient representative's text excerpts). Alternatively or in addition, the *summarize-normalize* paradigm according to an embodiment comprises mapping the clinically relevant sentences and concepts in the medical professional's 304 (e.g., radiation oncologist's) summary to standard clinical vocabulary (e.g., in the step S108 of converting the generated S106 verbatim report into the medical examination report).

As described in [10], the utterances 308-1; 308-2 from various medial examinations 302 (also denoted as patient-physician encounters) can be manually annotated and linked to specific sections and/or subsections in a subjective-objective-assessment-plan (SOAP) visit note, representing the medical examination report (also denotes as the document and/or summary), e.g., of a radiation oncology follow-up visit as an example of a medical examination 302.

For *locate* and *summarize* (e.g., as the steps of processing S104 the received S102 sensor data, generating S106 the verbatim report, and/or converting S108 the generated S106 verbatim report into the medical examination report), a sequence-to-sequence (seq2seq) language model (LM) (e.g., comprising bidirectional autoregressive transformers (BART) [11]) may be pretrained. The pre-training may be accomplished in a multi-modal setting (and/or using multiple sensors, in particular of at least two different types, and more particularly comprising a microphone 310 and a camera 312) with utterances-SOAP section and/or subsection as input alongside pixels and sensor time series which may represent additional vectors $q_i$ and $k_i$ used to train the LM.

Alternatively or in addition, a novel addition to the existing pre-trained seq2seq LM self-supervised objectives (e.g., token masking, token deletion, and/or sentence permutation) may be *query masking,* as exemplified in Fig. 5.

The LM, and/or the model (e.g., comprising, and/or corresponding to the trained, and/or learned, NN, AI, DL, and/or RL), according to an embodiment learns to predict the masked query 502 (and/or the assessment 504 thereof, e.g., at reference sign 508) in an input utterance-SOAP section/subsection pair 506 (and/or the mask 502), e.g., by minimizing a mask language modeling loss.

The pre-trained multi-modal seq2seq LM (mSeq2SeqPLM) may, according to an embodiment, be fine-tuned on a subset of medical examination 302 (e.g., radiation oncology) utterance-SOAP section/subsection data towards generating a preliminary medical examination report (also denoted as preliminary summary) focused mainly on the medical professional's 304 (e.g., radiation oncologist's) utterances 308-1 linked to SOAP sections/subsections.

According to some embodiments, the inventive technique may comprise post-processing of the medical examination reports (also denoted as meeting summaries). E.g., the post-processing may comprise the step of generating S108 the medical examination report from the verbatim report 318.

In an embodiment, each sentence in the preliminary medical examination report (also denoted as preliminary summary) may be given (and/or provided) as an input to a model for medical entity linking, e.g., Medlinker [12]. MedLinker is trained by finetuning a large language model with the MedMentions dataset [18]. The MedMentions dataset contains 4392 randomly selected PubMed papers and is annotated with mentions of the unified medical language system (UMLS) [19] entities. A little over 350000 entities are linked to concepts of 21 selected semantic types in UMLS. The MedLinker architecture uses a named entity recognition (NER) system producing mentions that are matched to entities using independent approaches based on n-grams and contextual embeddings, which are combined in a post-processing step into the final entity predictions. The named entity recognition system may comprise a conventional NER finetuning, where the last states of the language model are pooled and bidirectional long-short term memory-conditional random field (BiLSTM-CRF) may be applied on the pooled representation to generate entity spans. MedLinker uses three different types of matching algorithms to identify the final UMLS concept: firstly a zero-shot linking with approximate dictionary matching using 3-gram SimString matching, secondly linking by similarity to entity embeddings where the UMLS concepts are encoded using their concept and the last four layers are pooled, and thirdly training a minimal SoftMax classifier using contextual embeddings from large language models.

There exist several other entity linking systems that are trained on other corpus like BioCreative V CDR (BC5CDR) [20], National Center for Biotechnology Information (NCBI) [22], Cometa[23] and AskAPatient [24]. Notable systems include generative entity linking systems like GENRE [21] which uses generative language models to disambiguate entity mentions by auto-regressively generating the standard concept names conditioned on the inputs, GenBioEI [25] which achieves state-of-the-art entity linking performance on various biomedical entity linking datasets by generative methods, BioBART [26]-based entity linkers which have state-of-the-art performance on finetuning on entity linking datasets.

The entity linking model (e.g., embodied by the converting module 208, and/or assigned for performing the step S108 of converting the generated S106 verbatim report into the medical examination report) may be modified according to the inventive technique towards mapping sequences (e.g., beyond individual entities) to appropriate medical concepts (e.g., *blood in the urine caused by radiotherapy* may be normalized to *radiation-induced hematuria*). The entity linking model may leverage standard clinical ontologies like ICD10 and SNOMED-CT, such that the preliminary examination report (also denoted as preliminary summary) can be easily used for reporting of quality measures, and/or for use in future medical examinations 302 in the same, and/or a different (in particular, a related), medical field.

In free flow conversations (e.g., as typical for medical examinations 302), entity linking (e.g., performing the step S108 of converting the generated S106 verbatim report into the medical examination report)is a hard problem since it is difficult to identify the right context. For example, medical examinations 302 (also denoted as physician-patient conversations) may not, or need not, indicate specific areas of localization of certain symptoms, especially when the patient representative 306 (e.g., the patient) describes the symptoms ambiguously, and/or only indicated the localization by gestured.

Alternatively or in addition, there can be ambiguous spans of entities where the patient representative 306 (e.g., the patient) might be referring to multiple locations, and/or might be using (e.g., more) colloquial terms for the entities-The system 300 (and/or the device 200) may fail to understand the spans of the relevant entities.

Deep reinforcement learning (DRL)-based entity recognition and/or linking can become useful, e.g., on even a global scale, where the system 300 (and/or the computing device 200) learns to understand spans of several medical entities and/or the exact concept they are referring to. For each decoding step, the DRL algorithm may determine (e.g., compute) an utterance-level reward by comparing entities in the generated utterance (e.g., from the seq2seq LM) and the ground-truth entities (e.g., in the training data and/or training utterance) as shown in Eq. (2):

$$r = ROGUE(\{E_U\}, E_o) \qquad (2)$$
$$a = \Pi(r)$$

In Eq. (2), r is the reward, ROUGE (recall-oriented understudy for gisting evaluation) [17] refers to the n-gram overlaps of tokens and/or words between entities in the generated output ($E_u$) and those in the ground truth ($E_o$), $a$ is the action that should be taken (acceptance and/or rejection, e.g., by the entity linking model, of the generated entity), and $\pi$ is the policy network on which the actions are based.

In another embodiment, a medical examination report 302 (also denoted as summary) of patient representative 306 (e.g., patient) utterances 308-2 may be generated. Due to the fact that the subjective sections and/or subsections conventionally comprise patient narratives, such utterances 308-2 may be aggregated and provided to the patient as a form and/or text excerpts 322 (also denoted as patient utterance summary, and/or patient utterance summary form). Utterances from other sections and/or subsections (e.g., OAP) not represented in the preliminary summary generated by the fine-tuned mSeq2SeqPLM model can be included. The patient utterance summary form may comprise, e.g., a checkbox next to each text excerpt and/or utterance 308-2. The checkbox in the patient utterance summary form may the enable the patient representative 302 (e.g., the patient) to opt out of allowing the medical professional 306 (e.g., the radiation oncologist) access to specific utterances 308-2.

The patient utterance summary form, the text excerpted 322, and/or the one or more checkboxes may be provided by a digital twin of the patient (also denoted as patient digital twin app), e.g., on a patient representative's 306 electronic device.

According to an embodiment, the patient representative 306 (e.g., the patient) is able to view health-related metrics and/or related documents via the patient digital twin app. The patient digital twin app can serve as a comprehensive patient health record. The digital twin app may display data on the patient's daily activities (e.g., exercise, and/or sleep), past medical and/or surgical history, social history, vital signs, lab test results, and/or medical imaging. Alternatively or in addition, through the digital twin app, the patient representative 306 (e.g., the patient) may provide consent to which of his and/or her utterances 308-2 should be included with the verbatim report 318 (and/or the preliminary summary 326) is shown to the medical professional 304 (e.g., the radiation oncologist) after the medical examination 302 (also denoted as visit). The opt-out decision may only be possible for a predetermined period of time after the medical examination 302, and/or visit (e.g., for two days). If the predetermined period of time elapses without the patient representative 306 (e.g., the patient) selecting the checkboxes to accept, and/or omit, one or more utterances 308-2, according to an embodiment all utterances will be include in the preliminary medical examination report (also: preliminary summary).

In another embodiment, utterances in the verbatim report 318, and/or in a preliminary medical examination report (also: preliminary summary) 322; 326, may refer to, e.g., specific, medical imaging (also denoted as medical images in the context of the inventive technique, and/or, e.g., comprising one or more CT scans, MRI scans, and/or Ultrasound scans) taken on one or more dates, in particular preceding the medical examination 302. The utterances 308-1; 308-2 in the verbatim report 318 may be analyzed by the entity linking model. The extracted results (e.g., imaging modality, site and/or date) may be used as an input query to a radiation oncology picture archiving and communication system (PACS), leading to retrieval of the medical images. The medical images may then be attached to the preliminary medical examination report 322; 326 (also: preliminary summary).

In another embodiment, the verbatim report 318, and/or the (e.g., preliminary) summary 322; 324(e.g., without and/or with medical images) with the consented patient utterances are made available (e.g., in the method step S114) to the medical professional 304 (e.g., radiation oncologist) for verification. The medical professional 304 (e.g., radiation oncologist) can then review, and/or edit, the (e.g., preliminary) summary (and/or have another review of the medical images if needed) until he and/or she determines that the e.g., preliminary) summary is satisfactory. Then the medical professional 304 (e.g., radiation oncologist) may (e.g., as the method step S116) click a checkbox stating *verification completed.* He and/or she may then copy and paste (e.g., as the method step S118) the verified summary and/or medical examination report directly into the oncology information system (OIS) and/or EMR, e.g., as the final follow-up visit note.

The inventive technique may comprise end-to-end learning. According to an embodiment, an interactive AI system (e.g., involving feedback from the medical professional 306, in particular radiation oncologist, in the steps S114 and S116, and optionally to some extent also from the patient representative 306, in the steps S110 and S112) may comprise training the, e.g., fine-tuned, mSeq2SeqPLM using reinforcement learning (RL) using a loss function, e.g., with rewards, computed out of the feedback [13, 14, 15].

The impact of other post-processing steps, such as entity recognition and/or linking component [16], may also be included in the loss function, e.g., reward computation. The corrections made by the medical professional 304 (e.g., the radiation oncologist) on the extracted entities in the summaries (and/or the, in particular preliminary, medical examination report 326) may force mSeq2SeqPLM to generate medical examination reports (also: summaries) with richer context, e.g., compared to conventional speech-recognition based techniques.

The most important takeaways from a medical examination 302 (also: physician-patient conversation) may often dependent be on the medical professional 304 (e.g., physician's) sub-specialty. In the exemplary case of radiation oncology, the reinforcement learning based systems 300 (and/or computing device 200) may focus on learning, e.g., on a local scale for a designated practice group, medical facility (also: hospital), and/or sub-specialty to provide more relevant medical examination reports (also: summaries), e.g., compared to conventional speech-recognition based techniques. In an embodiment, the RL agent (e.g., mSeq2SeqPLM) may be further customized using the attributes (e.g., designated practice group, medical facility, and/or sub-specialty) as, e.g., additional and/or supplementary, features. The RL agent may be fine-tuned, e.g., only, for close-domain (e.g., the sub-specialty and neighboring the sub-specialty) utterances-SOAP section and/or subsection pairs.

Alternatively or in addition, the inventive technique may comprise privacy by design. In an embodiment, once the *verification completed* box is checked (e.g., in the step of receiving S116 the user input by the medical professional 304), the sensor data 316 - which may also be denoted as original verbal (and/or non-verbal) recordings - used to generate the medical examination report (also: summary), which may have been stored, e.g., in the medical facility's (also: healthcare institution's) private cloud governed by enterprise-level data privacy agreements, may be permanently deleted, as indicated at reference sign 328 in Fig. 3.

If the verification completed box is not checked within a predetermined period of time (which may be independently determined from the predetermined period of time for the user input by the patient representative 306) after the medical examination 302, and/or visit, (e.g., two days), according to an embodiment the preliminary medical examination report (also: preliminary summary) may be permanently deleted 328, e.g., in addition to the sensor data 316 (also: original recordings). According to another embodiment, which is combinable with the other embodiments, only the patient utterance summary 322 (also denoted as the patient representative's text excerpts) accessible via the patient digital twin app may persist. The patient utterance summary 322 may be retained and/or deleted at the discretion of the patient. While these strict data deletion measures would contribute towards preventing unauthorized access to protected health information (PHI) in the cloud, oncology information system (OIS), HER, and/or EMR, the patient representative 306 (e.g., the patient) may be simultaneously empowered to be responsible for protection of his, and/or her, utterances (also: narratives) 308-2 provided during the medical examination 302 (also: follow-up visit).

The inventive technique can be distinguished from alternative methods for automated documentation in radiation oncology based on one or more of the following features. The inventive technique may be multi-modal (e.g., comprising audio and/or text, video and/or images, including medical imaging, in particular medical imaging discussed during the medical examination 302), and/or multi-participant based visit notes can be generated by pre-training a (e.g., seq2seq) LM. Alternatively or in addition, the inventive technique may comprise the *summarize-normalize* paradigm utilizing the pretraining and fine-tuning of a multi-modal (e.g., seq2seq) LM (e.g., with an additional training objective of *query masking*). Further alternatively or in addition, the inventive technique may make use of medical entity linking (e.g., comprising mapping sequences to standardized medical concepts). A score may be used as reward, and/or loss function, for a reinforcement learning based end-to-end training of a pre-trained (e.g., seq2seq) LM. Further alternatively or in addition, the inventive technique may integrate a patient representative's (and/or patient's) consent for access to specific patient narratives via the opt-out checkboxes on the patient utterance summary form. Further alternatively or in addition, the inventive technique may comprise deleting 328 the original verbal and/or non-verbal recordings once *verification completed* has been checked by the radiation oncologist, and/or when a certain time period after the visit has elapsed. Further alternatively or in addition, the inventive technique may make use of RL-based system that can help personalize the automated documentation of visit notes.

Wherever not already described explicitly, individual embodiments, or their individual aspects and features, described in relation to the drawings can be combined or exchanged with one another without limiting or widening the scope of the described invention, whenever such a combination or exchange is meaningful and in the sense of this invention. Advantages which are described with respect to a particular embodiment of present invention or with respect to a particular figure are, wherever applicable, also advantages of other embodiments of the present invention.

List of Cited Documents

[2] Moy, A.J., Schwartz, J.M., Chen, R., Sadri, S., Lucas, E., Cato, K.D. and Rossetti, S.C., 2021. Measurement of clinical documentation burden among physicians and nurses using electronic health records: a scoping review. Journal of the American Medical Informatics Association, 28(5), pp.998-1008.

[3] Lin, S., 2020, May. The present and future of team documentation: the role of patients, families, and artificial intelligence. In Mayo Clinic Proceedings (Vol. 95, No. 5, pp. 852-855). Elsevier.

[4] Lutz, C. and Newlands, G., 2021. Privacy and smart speakers: A multi-dimensional approach. The Information Society, 37(3), pp.147-162.

[4] Barth, S., Ionita, D. and Hartel, P., 2022. Understanding Online Privacy-A Systematic Review of Privacy Visualizations and Privacy by Design Guidelines. ACM Computing Surveys (CSUR), 55(3), pp.1-37.

[5] Huebner, A., Ji, W. and Xiao, X., 2021. Meeting Summarization with Pre-training and Clustering Methods. arXiv preprint arXiv:2111.08210.

[6] Dela Cruz, J.E., Shabosky, J.C., Albrecht, M., Clark, T.R., Milbrandt, J.C., Markwell, S.J. and Kegg, J.A., 2014. Typed versus voice recognition for data entry in electronic health records: emergency physician time use and interruptions. Western Journal of Emergency Medicine, 15(4), p.541.

[7] Hodgson, T., Magrabi, F. and Coiera, E., 2017. Efficiency and safety of speech recognition for documentation in the electronic health record. Journal of the American Medical Informatics Association, 24(6), pp.1127-1133.

[8] Molenaar, S., Maas, L., Burriel, V., Dalpiaz, F. and Brinkkemper, S., 2020, June. Medical dialogue summarization for automated reporting in healthcare. In International Conference on Advanced Information Systems Engineering (pp. 76-88). Springer, Cham.

[9] Zhu, H., Dong, L., Wei, F., Qin, B. and Liu, T., 2022. Transforming wikipedia into augmented data for query-focused summarization. IEEE/ACM Transactions on Audio, Speech, and Language Processing.

[10] Krishna, K., Pavel, A., Schloss, B., Bigham, J.P. and Lipton, Z.C., 2021. Extracting structured data from physician-patient conversations by predicting noteworthy utterances. In Explainable AI in Healthcare and Medicine (pp. 155-169). Springer, Cham.

[11] Lewis, M., Liu, Y., Goyal, N., Ghazvininejad, M., Mohamed, A., Levy, O., Stoyanov, V. and Zettlemoyer, L.,

2019. Bart: Denoising sequence-to-sequence pre-training for natural language generation, translation, and comprehension. arXiv preprint arXiv:1910.13461.

[12] Loureiro, D. and Jorge, A.M., 2020, April. Medlinker: Medical entity linking with neural representations and dictionary matching. In European Conference on Information Retrieval (pp. 230-237). Springer, Cham.

[13] Alomari, Ayham, et al. "Deep reinforcement and transfer learning for abstractive text summarization: A review." Computer Speech & Language 71 (2022): 101276.

[14] Zhang, Mengli, et al. "FAR-ASS: Fact-aware reinforced abstractive sentence summarization." Information Processing & Management 58.3 (2021): 102478.

[15] Xu, Tianyang, and Chunyun Zhang. "Reinforced Generative Adversarial Network for Abstractive Text Summarization." arXiv preprint arXiv:2105.15176 (2021).

[16] Yang, Yaosheng, et al. "Distantly supervised NER with partial annotation learning and reinforcement learning." Proceedings of the 27th International Conference on Computational Linguistics. 2018.

[17] Lin, C.Y., 2004, July. Rouge: A package for automatic evaluation of summaries. In Text summarization branches out (pp. 74-81).

[18] Sunil Mohan, Rico Angell, Nicholas Monath, Andrew McCallum. 2021. Low Resource Recognition and Linking of Biomedical Concepts from a Large Ontology. In Proceedings of the ACM Conference on Bioinformatics, Computational Biology, and Health Informatics (ACM-BCB), 2021.

[19] Olivier Bodenreider. 2004. The unified medical language system (umls): integrating biomedical terminology. Nucleic acids research, 32 Database issue:D267-70.

[20] Jiao Li, Yueping Sun, Robin J Johnson, Daniela Sciaky, Chih-Hsuan Wei, Robert Leaman, Allan Peter Davis, Carolyn J Mattingly, Thomas C Wiegers, and Zhiyong Lu. 2016. Biocreative v cdr task corpus: a resource for chemical disease relation extraction. Database, 2016.

[21] Nicola De Cao, Gautier Izacard, Sebastian Riedel, and Fabio Petroni. 2021a. Autoregressive entity retrieval. In International Conference on Learning Representations.

[22] Rezarta Islamaj Dogan, Robert Leaman, and Zhiyong Lu. 2014. Ncbi disease corpus: a resource for disease name recognition and concept normalization. Journal of biomedical informatics, 47:1-10.

[23] Marco Basaldella, Fangyu Liu, Ehsan Shareghi, and Nigel Collier. 2020. COMETA: A corpus for medical entity linking in the social media. In Proceedings of the 2020 Conference on Empirical Methods in Natural Language Processing (EMNLP), pages 3122-3137, Online. Association for Computational Linguistics.

[24] Yuan, Hongyi, Zheng Yuan, and Sheng Yu. "Generative Biomedical Entity Linking via Knowledge Base-Guided Pre-training and Synonyms-Aware Fine-tuning." arXiv preprint arXiv:2204.05164 (2022).

[25] Yuan, Hongyi, et al. "Biobart: pretraining and evaluation of a biomedical generative language model." arXiv preprint arXiv:2204.03905 (2022).

List of Reference Signs

| | |
|---|---|
| 100 | Method |
| S102 | Step of receiving sensor data |
| S104 | Step of processing the received sensor data |
| S106 | Step of generating a verbatim report |
| S108 | Step of converting the generated verbatim report into a medical examination report |
| S110 | Step of providing text excerpts to a patient representative for approval |
| S112 | Step of receiving a user input by the patient representative |
| S114 | Step of providing text excerpts to a medical professional for verification |
| S116 | Step of receiving a user input by the medical professional |
| S118 | Step of storing the medical examination report in an electronic medical report database |
| S120 | Step of outputting the stored medical examination report |
| 200 | Computing device |
| 202 | Sensor interface |
| 204 | Processing module |
| 206 | Generating module |
| 208 | Converting module |
| 210 | 1st text excerpts sending interface |
| 212 | 1st receiving user input interface |
| 214 | 2nd text excerpts sending interface |
| 216 | 2nd receiving user input interface |

| 218 | Storage interface |
|---|---|
| 220 | Output interface |
| 222; | Memory |
| 222' 226 | Processing unit, in particular CPU |
| 228; | External interface |
| 228' 302 | Medical examination |
| 304 | Medical professional |
| 306 | Patient |
| 308-1 | Utterance by medical professional |
| 308-2 | Utterance by patient |
| 310 | 1st sensor, in particular microphone |
| 312 | 2nd sensor, in particular video camera |
| 314 | Cloud |
| 316 | Sensor data, in particular comprising audio data |
| 318 | Verbatim report |
| 320 | Multi-modal summary program |
| 322 | Text excerpts assigned to a patient representative |
| 324 | Normalization program |
| 326 | Text excerpts assigned to a medical professional |
| 328 | Deleting data from a cloud |
| 402 | Input embedding to decoder, optionally comprising positional embedding |
| 404 | Self-attention layer |
| 405 | Encoder-decoder attention layer |
| 406 | Masked multi-head |
| 408-1; | Add and normalize layer |
| 408-2; 408-3 410 | Multi-head |
| 412 | Feed forward |
| 413 | Video encoder |
| 414 | 1st decoder |
| 416-1; | Connections between layers |
| 416-2; 416-3 418 | 2nd decoder |
| 420 | Linear layer |
| 422 | SoftMax layer |
| 424 | Input query to SoftMax layer |
| 426 | Output of NN architecture, in particular medical examination report |
| 428 | Video encoder output |
| 430 | Feed from video encoder output to decoders |
| 502 | Mask |
| 504 | Assessment |
| 506 | Input utterance-SOAP section/subsection pair to mask |
| 508 | Output, in particular based on the mask input |

**Claims**

1. Computer implemented method (100) for generating a medical examination report from sensor data (316) of a medical examination, comprising the method steps of:

- Receiving (S102) sensor data (316) from a set of sensors (310; 312) in relation to a medical examination (302), wherein the set of sensors (310; 312) comprises at least one microphone (310) and the sensor data (316) comprise at least audio data (316), wherein the audio data (316) comprise utterances (308-1; 308-2) by a medical professional (304) and a patient representative (306) participating in the medical examination (302);
- Processing (S104) the received (S102) sensor data (316) according to the type of sensors (310; 312), wherein the processing (S104) comprises at least transforming the audio data (316) into text (318);
- Generating (S106) a verbatim report (318) of the medical examination based on the processed (S104) sensor data (316), wherein the verbatim report (318) comprises each excerpt (322; 326) of the text (318) being assigned to the medical professional (304) or the patient representative (306);
- Converting (S108) the generated (S106) verbatim report (318) into a medical examination report, wherein the medical examination report comprises a summary of the verbatim report (318), wherein the summary comprises vocabulary, and/or text excerpts (322; 326), by accessing a predetermined ontology database (320) in relation to a medical field of the medical examination; and
- Storing (S118) the medical examination report in an electronic medical report database.

2. Method (100) according to claim 1, wherein the set of sensors (310; 312) comprises at least one further sensor selected from the group of:

   - A camera, in particular a video camera; and
   - A motion sensor.

3. Method (100) according to any of the preceding claims, further comprising the method steps of:

   - Providing (S110) the text excerpts (322) of the converted (S108) medical examination report, which are assigned to the patient representative (306), to the patient representative (306) for approval; and
   - Receiving (S112) a user input by the patient representative (306), wherein the user input is indicative of approval, and/or rejection, in particular of the correctness, of the provided (S110) text excerpts (322) assigned to the patient representative (306).

4. Method (100) according to any of the preceding claims, further comprising the method steps of:

   - Providing (S114) the text excerpts (326) of the converted (S108) medical examination report, which are assigned to the medical professional (304), to the medical professional (304) for verification; and
   - Receiving (S116) a user input, by the medical professional (304), indicative of a validation of the provided (S114) text excerpts (326) assigned to the medical professional (304).

5. Method (100) according to any of the preceding claims, further comprising the method steps of:

   - Temporarily storing intermediate data in a private cloud (314), wherein the intermediate data comprise the received sensor data (316), the generated verbatim report (318) and/or at least parts of the converted medical examination report (322; 326); and
   - Deleting the temporarily stored intermediate data from the private cloud (314) after a predetermined period of time, after a rejection by the patient representative, and/or after a verification by the medical professional.

6. Method (100) according to any of the preceding claims, wherein storing (S118) the medical examination report in an electronic medical report database comprises storing (S118) the medical examination report in a centralized memory of a medical facility, in particular of the medical facility where the medical examination was performed.

7. Method (100) according to any of the preceding claims, further comprising the method step of:

   - Outputting (S120) the stored (S118) medical examination report.

8. Method (100) according to any of the preceding claims, wherein storing (S118) the medical examination report in an electronic medical report database comprises storing (S118) the medical examination report in a digital twin of the patient, and/or wherein the providing (S110) of the text excerpts (322) assigned to the patient representative (306) and the receiving (S112) of the user input by the patient representative according to claim 3 are performed using the digital twin of the patient.

9. Method (100) according to any of the preceding claims, wherein the assignment of each excerpt (322; 326) of the text (318) to the medical professional (304) or the patient representative (306) in the step of generating (S106) a verbatim report (318) is based on at least one of:

   - Filtering of the audio data (316) according to a frequency pattern, and/or according to a low pass filter, and/or a high pass filter, wherein the filtered audio data (316) are classified according to characteristics determined by the filtering, and wherein the classifying characteristics are assigned to the medical professional (304) and/or the patient representative (306);
   - Extracting time stamps from the audio data (316) and further sensor data, combining the audio data (316) and the further sensor data, in particular visual data, according to the extracted time stamps, and assigning each excerpt (322; 326) of the text (318) to the medical professional (304) or the patient representative (306) according to the combination of the audio data (316) and the further sensor data, in particular the visual data, per time stamp.

10. Method (100) according to any of the preceding claims, further comprising the method steps of:

- Identifying, based on the processed (S104) sensor data (316), medical imaging data that were examined during the medical examination; and
- Appending the medical imaging data to the medical examination report.

11. Method (100) according to any of the preceding claims, wherein converting (S108) the generated (S106) verbatim report (318) into a medical examination report is based on a trained artificial intelligence, AI, a trained neural network, NN, deep learning, DL, and/or reinforcement learning, RL.

12. Method (100) according to the directly preceding claim, wherein the training of the AI and/or the NN, the DL, and/or the RL is based on training data sets, wherein each training data set comprises sensor data (316) and an associated, in particular at least manually compiled, medical examination report.

13. Method (100) according to claim 11 or 12, wherein the training, and/or learning, is based on optimizing a loss function.

14. Method (100) according to any of the preceding claims, wherein the medical examination comprises a radiology, and/or an, in particular radiological, oncology examination.

15. Computing device (200) for generating a medical examination report from sensor data (316) of a medical examination, comprising:

- A sensor interface (202) configured to receive sensor data (316) from a set of sensors (310; 312) in relation to a medical examination (302), wherein the set of sensors (310; 312) comprises at least one microphone (310) and the sensor data (316) comprise at least audio data (316), wherein the audio data (316) comprise utterances (308-1; 308-2) by a medical professional (304) and a patient representative (306) participating in the medical examination (302);
- A processing module (204) configured to process the received sensor data (316) according to the type of sensors (310; 312), wherein the processing comprises at least transforming the audio data (316) into text (318);
- A generating unit (206) configured to generate a verbatim report (318) of the medical examination based on the processed sensor data (316), wherein the verbatim report (318) comprises each excerpt (322; 326) of the text (318) being assigned to the medical professional (304) or the patient representative (306);
- A converting module (208) configured to convert the generated verbatim report (318) into a medical examination report, wherein the medical examination report comprises a summary of the verbatim report (318), wherein the summary comprises vocabulary, and/or text excerpts (322; 326), by accessing a predetermined ontology database (320) in relation to a medical field of the medical examination; and
- A storage interface (218) configured to forward the medical examination report to an electronic medical report database for storing.

16. System for generating a medical examination report from sensor data (316) of a medical examination, comprising:

- A set of sensors (310; 312) comprising at least one microphone (310), wherein the set of sensors (310; 312) is configured to capture sensor data (316) relating to a medical professional (304) and a patient representative (306) participating in the medical examination (302), wherein the sensor data (316) comprise at least audio data (316) captured by the at least one microphone (310), wherein the audio data (316) comprise utterances (308-1; 308-2) by the medical professional (304) and the patient representative (306);
- A processing module (204) configured to process the sensor data (316) according to the type of sensors (310; 312), wherein the processing comprises at least transforming the audio data (316) into text (318);
- A generating module (206) configured to generate a verbatim report (318) of the medical examination based on the processed sensor data (316), wherein the verbatim report (318) comprises each excerpt (322; 326) of the text (318) being assigned to the medical professional (304) or the patient representative (306);
- A converting module (208) configured to convert the generated verbatim report (318) into a medical examination report, wherein the medical examination report comprises a summary of the verbatim report (318), wherein the summary comprises vocabulary, and/or text excerpts (322; 326);
- An ontology database (320) in relation to a medical field of the medical examination, wherein the ontology database (320) comprises the vocabulary, and/or the text excerpts (322; 326) for the summary of the verbatim report (318), and wherein the ontology database (320) is configured to be accessed for converting the generated verbatim report (318) into the medical examination report; and
- A storage module (222) configured to store the medical examination report in an electronic medical report database.

17. A computer program comprising program elements which induce a computing device (200) to carry out the steps of the method for generating a medical examination report from sensor data (316) of a medical examination according to one of the preceding method claims, when the program elements are loaded into a memory (222) of the computing device (200).

18. A computer-readable medium on which program elements are stored that can be read and executed by a computing device (200), in order to perform steps of the method for generating a medical examination report from sensor data (316) of a medical examination according to one of the preceding method claims, when the program elements are executed by the computing device (200).


**Amended claims in accordance with Rule 137(2) EPC.**

1. Computer implemented method (100) for generating a medical examination report from sensor data (316) of a medical examination, comprising the method steps of:

   - Receiving (S102) sensor data (316) from a set of sensors (310; 312) in relation to a medical examination (302), wherein the set of sensors (310; 312) comprises at least one microphone (310) and the sensor data (316) comprise at least audio data (316), wherein the audio data (316) comprise utterances (308-1; 308-2) by a medical professional (304) and a patient representative (306) participating in the medical examination (302), wherein the set of sensors (310; 312) further comprises a camera, in particular a video camera;
   - Processing (S104) the received (S102) sensor data (316) according to the type of sensors (310; 312), wherein the processing (S104) comprises at least transforming the audio data (316) into text (318);
   - Generating (S106) a verbatim report (318) of the medical examination based on the processed (S104) sensor data (316), wherein the verbatim report (318) comprises each excerpt (322; 326) of the text (318) being assigned to the medical professional (304) or the patient representative (306), wherein the assignment of each excerpt (322; 326) of the text (318) to the medical professional (304) or the patient representative (306) in the step of generating (S106) a verbatim report (318) is based on extracting time stamps from the audio data (316) and further sensor data, combining the audio data (316) and the further sensor data, namely visual data, according to the extracted time stamps, and assigning each excerpt (322; 326) of the text (318) to the medical professional (304) or the patient representative (306) according to the combination of the audio data (316) and the further sensor data, namely the visual data, per time stamp;
   - Converting (S108) the generated (S106) verbatim report (318) into a medical examination report, wherein the medical examination report comprises a summary of the verbatim report (318), wherein the summary comprises vocabulary, and/or text excerpts (322; 326), by accessing a predetermined ontology database (320) in relation to a medical field of the medical examination;
   - Providing (S110) the text excerpts (322) of the converted (S108) medical examination report, which are assigned to the patient representative (306), to the patient representative (306) for approval; and
   - Receiving (S112) a user input by the patient representative (306), wherein the user input is indicative of approval, and/or rejection, in particular of the correctness, of the provided (S110) text excerpts (322) assigned to the patient representative (306); and
   - Storing (S118) the medical examination report in an electronic medical report database.

2. Method (100) according to claim 1, wherein the set of sensors (310; 312) further comprises a motion sensor.

3. Method (100) according to any of the preceding claims, further comprising the method steps of:

   - Providing (S114) the text excerpts (326) of the converted (S108) medical examination report, which are assigned to the medical professional (304), to the medical professional (304) for verification; and
   - Receiving (S116) a user input, by the medical professional (304), indicative of a validation of the provided (S114) text excerpts (326) assigned to the medical professional (304).

4. Method (100) according to any of the preceding claims, further comprising the method steps of:

- Temporarily storing intermediate data in a private cloud (314), wherein the intermediate data comprise the received sensor data (316), the generated verbatim report (318) and/or at least parts of the converted medical examination report (322; 326); and
- Deleting the temporarily stored intermediate data from the private cloud (314) after a predetermined period of time, after a rejection by the patient representative, and/or after a verification by the medical professional.

5. Method (100) according to any of the preceding claims, wherein storing (S118) the medical examination report in an electronic medical report database comprises storing (S118) the medical examination report in a centralized memory of a medical facility, in particular of the medical facility where the medical examination was performed.

6. Method (100) according to any of the preceding claims, further comprising the method step of:

- Outputting (S120) the stored (S118) medical examination report.

7. Method (100) according to any of the preceding claims, wherein storing (S118) the medical examination report in an electronic medical report database comprises storing (S118) the medical examination report in a digital twin of the patient, and/or wherein the providing (S110) of the text excerpts (322) assigned to the patient representative (306) and the receiving (S112) of the user input by the patient representative according to claim 3 are performed using the digital twin of the patient.

8. Method (100) according to any of the preceding claims, wherein the assignment of each excerpt (322; 326) of the text (318) to the medical professional (304) or the patient representative (306) in the step of generating (S106) a verbatim report (318) is further based on:

- Filtering of the audio data (316) according to a frequency pattern, and/or according to a low pass filter, and/or a high pass filter, wherein the filtered audio data (316) are classified according to characteristics determined by the filtering, and wherein the classifying characteristics are assigned to the medical professional (304) and/or the patient representative (306).

9. Method (100) according to any of the preceding claims, further comprising the method steps of:

- Identifying, based on the processed (S104) sensor data (316), medical imaging data that were examined during the medical examination; and
- Appending the medical imaging data to the medical examination report.

10. Method (100) according to any of the preceding claims, wherein converting (S108) the generated (S106) verbatim report (318) into a medical examination report is based on a trained artificial intelligence, AI, a trained neural network, NN, deep learning, DL, and/or reinforcement learning, RL.

11. Method (100) according to the directly preceding claim, wherein the training of the AI and/or the NN, the DL, and/or the RL is based on training data sets, wherein each training data set comprises sensor data (316) and an associated, in particular at least manually compiled, medical examination report.

12. Method (100) according to claim 10 or 11, wherein the training, and/or learning, is based on optimizing a loss function.

13. Method (100) according to any of the preceding claims, wherein the medical examination comprises a radiology, and/or an, in particular radiological, oncology examination.

14. Computing device (200) for generating a medical examination report from sensor data (316) of a medical examination, comprising:

- A sensor interface (202) configured to receive sensor data (316) from a set of sensors (310; 312) in relation to a medical examination (302), wherein the set of sensors (310; 312) comprises at least one microphone (310) and the sensor data (316) comprise at least audio data (316), wherein the audio data (316) comprise utterances (308-1; 308-2) by a medical professional (304) and a patient representative (306) participating in the medical examination (302), wherein the set of sensors (310; 312) further comprises a camera, in particular a video camera;

- A processing module (204) configured to process the received sensor data (316) according to the type of sensors (310; 312), wherein the processing comprises at least transforming the audio data (316) into text (318);

- A generating module (206) configured to generate a verbatim report (318) of the medical examination based on the processed sensor data (316), wherein the verbatim report (318) comprises each excerpt (322; 326) of the text (318) being assigned to the medical professional (304) or the patient representative (306), wherein the assignment of each excerpt (322; 326) of the text (318) to the medical professional (304) or the patient representative (306) in the step of generating (S106) a verbatim report (318) is based on extracting time stamps from the audio data (316) and further sensor data, combining the audio data (316) and the further sensor data, namely visual data, according to the extracted time stamps, and assigning each excerpt (322; 326) of the text (318) to the medical professional (304) or the patient representative (306) according to the combination of the audio data (316) and the further sensor data, namely the visual data, per time stamp;

- A converting module (208) configured to convert the generated verbatim report (318) into a medical examination report, wherein the medical examination report comprises a summary of the verbatim report (318), wherein the summary comprises vocabulary, and/or text excerpts (322; 326), by accessing a predetermined ontology database (320) in relation to a medical field of the medical examination;

- A first text excerpts sending interface (210) configured for providing the text excerpts (322) of the converted medical examination report, which are assigned to the patient representative (306), to the patient representative (306) for approval;

- A first user input receiving interface (212) configured for receiving a user input by the patient representative (306), wherein the user input is indicative of approval, and/or rejection, in particular of the correctness, of the provided text excerpts (322) assigned to the patient representative (306); and

- A storage interface (218) configured to forward the medical examination report to an electronic medical report database for storing.

15. System for generating a medical examination report from sensor data (316) of a medical examination, comprising:

- A set of sensors (310; 312) comprising at least one microphone (310) and a camera, in particular a video camera, wherein the set of sensors (310; 312) is configured to capture sensor data (316) relating to a medical professional (304) and a patient representative (306) participating in the medical examination (302), wherein the sensor data (316) comprise at least audio data (316) captured by the at least one microphone (310), wherein the audio data (316) comprise utterances (308-1; 308-2) by the medical professional (304) and the patient representative (306);

- A processing module (204) configured to process the sensor data (316) according to the type of sensors (310; 312), wherein the processing comprises at least transforming the audio data (316) into text (318);

- A generating module (206) configured to generate a verbatim report (318) of the medical examination based on the processed sensor data (316), wherein the verbatim report (318) comprises each excerpt (322; 326) of the text (318) being assigned to the medical professional (304) or the patient representative (306), wherein the assignment of each excerpt (322; 326) of the text (318) to the medical professional (304) or the patient representative (306) in the step of generating (S106) a verbatim report (318) is based on extracting time stamps from the audio data (316) and further sensor data, combining the audio data (316) and the further sensor data, namely visual data, according to the extracted time stamps, and assigning each excerpt (322; 326) of the text (318) to the medical professional (304) or the patient representative (306) according to the combination of the audio data (316) and the further sensor data, namely the visual data, per time stamp;

- A converting module (208) configured to convert the generated verbatim report (318) into a medical examination report, wherein the medical examination report comprises a summary of the verbatim report (318), wherein the summary comprises vocabulary, and/or text excerpts (322; 326);

- A first text excerpts sending interface (210) configured for providing the text excerpts (322) of the converted medical examination report, which are assigned to the patient representative (306), to the patient representative (306) for approval;

- A first user input receiving interface (212) configured for receiving a user input by the patient representative (306), wherein the user input is indicative of approval, and/or rejection, in particular of the correctness, of the provided text excerpts (322) assigned to the patient representative (306);

- An ontology database (320) in relation to a medical field of the medical examination, wherein the ontology database (320) comprises the vocabulary, and/or the text excerpts (322; 326) for the summary of the verbatim report (318), and wherein the ontology database (320) is configured to be accessed for converting the generated verbatim report (318) into the medical examination report; and

- A storage module (222) configured to store the medical examination report in an electronic medical report database.

16. A computer program comprising program elements which induce a computing device (200) to carry out the steps of the method for generating a medical examination report from sensor data (316) of a medical examination according to one of the preceding method claims, when the program elements are loaded into a memory (222) of the computing device (200).

17. A computer-readable medium on which program elements are stored that can be read and executed by a computing device (200), in order to perform steps of the method for generating a medical examination report from sensor data (316) of a medical examination according to one of the preceding method claims, when the program elements are executed by the computing device (200).

FIG 1

# FIG 2

FIG 3

EP 4 421 816 A1

FIG 4

Video Encoder — 413

encoder output — 428

302

306    304

EP 4 421 816 A1

426

424

422

Softmax

Linear — 420

430

DECODER #2 — 418

416-3 — Add & Normalize — 408-3

412 — Feed Forward    Feed Forward — 412

Add & Normalize — 408-2

Encoder-Decoder Attention    Multi-head — 410

416-2 — 405

Add & Normalize — 408-1

416-1 — Self-Attention    Masked Multi-head — 406

414 — DECODER #1 — 404

402 — ⊕    ⊕ — 402

308-1; 308-2

27

FIG 5

508

504

I think the blood you have been noticing in your urine may be caused by radiation from your therapy

Assessment

I think the blood you have been noticing in your urine may be caused by radiation from your therapy

MASK

506

502

EP 4 421 816 A1

Europäisches
Patentamt

European
Patent Office

Office européen
des brevets

# EUROPEAN SEARCH REPORT

Application Number

**EP 23 15 7914**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MAAS LIENTJE ET AL: "The Care2Report System: Automated Medical Reporting as an Integrated Solution to Reduce Administrative Burden in Healthcare", PROCEEDINGS OF THE 53RD HAWAII INTERNATIONAL CONFERENCE ON SYSTEM SCIENCES, 7 January 2020 (2020-01-07), pages 3608-3617, XP93063688, ISSN: 2572-6862, DOI: 10.24251/HICSS.2020.442 ISBN: 978-0-9981331-3-3 Retrieved from the Internet: URL:https://scholarspace.manoa.hawaii.edu/ server/api/core/bitstreams/39916eed-1e7f-4 fa9-9e41-40599f4042c2/content> | 1-5,7, 15-18 | INV. G16H15/00 |
| Y | * Abstract; Chapter 3, "The Care2Report system"; Figure 1 * ----- -/-- | 6,8-13 | |

| | | | TECHNICAL FIELDS SEARCHED (IPC) |
|---|---|---|---|
| | | | G16H |

The present search report has been drawn up for all claims

| Place of search | Date of completion of the search | Examiner |
|---|---|---|
| Munich | 13 July 2023 | Schmidt, Karsten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 3**

EUROPEAN SEARCH REPORT

Application Number

EP 23 15 7914

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Molenaar Sabine ET AL: "Medical Dialogue Summarization for Automated Reporting in Healthcare : CAiSE 2020 International Workshops, Grenoble, France, June 8-12, 2020, Proceedings" In: "Advanced Information Systems Engineering Workshops : CAiSE 2020 International Workshops, Grenoble, France, June 8-12, 2020, Proceedings", 1 January 2020 (2020-01-01), Springer International Publishing, Cham, XP93063694, ISSN: 1865-1348 ISBN: 978-3-030-49165-9 vol. 382, pages 76-88, DOI: 10.1007/978-3-030-49165-9_7, Retrieved from the Internet: URL:http://link.springer.com/content/pdf/10.1007/978-3-030-49165-9_7> | 1-5,7, 15-18 | |
| Y | * Abstract; Chapter 1, "Introduction"; Chapter 3, "Dialogue Summarization Pipeline"; figure 1 * | 6,8-13 | TECHNICAL FIELDS SEARCHED (IPC) |
| | ----- | | |
| Y | US 2013/054271 A1 (LANGFORD JESSICA JOAN [US] ET AL) 28 February 2013 (2013-02-28) * paragraph [0020] * | 6 | |
| | ----- | | |
| Y | US 2023/051982 A1 (SASIDHARAN SANAND [IN] ET AL) 16 February 2023 (2023-02-16) * paragraph [0022] * | 8 | |
| | ----- | | |
| Y | CN 105 260 974 A (QILU CHILDREN S HOSPITAL OF SHANDONG UNIVERSITY) 20 January 2016 (2016-01-20) * paragraph [0067] * | 9 | |
| | ----- | | |
| | -/-- | | |

The present search report has been drawn up for all claims

| Place of search | Date of completion of the search | Examiner |
|---|---|---|
| Munich | 13 July 2023 | Schmidt, Karsten |

EPO FORM 1503 03.82 (P04C01)

page 2 of 3

Europäisches
Patentamt

European
Patent Office

Office européen
des brevets

# EUROPEAN SEARCH REPORT

Application Number

EP 23 15 7914

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2007/288268 A1 (WEEKS WALTER L [US]) 13 December 2007 (2007-12-13) * claim 20 * ----- | 10 | |
| Y | NEEL KANWAL ET AL: "Attention-based Clinical Note Summarization", ARXIV.ORG, CORNELL UNIVERSITY LIBRARY, 201 OLIN LIBRARY CORNELL UNIVERSITY ITHACA, NY 14853, 1 October 2021 (2021-10-01), XP091066844, * the whole document * ----- | 11-13 | |

| | | TECHNICAL FIELDS SEARCHED (IPC) |
|---|---|---|

The present search report has been drawn up for all claims

| Place of search | Date of completion of the search | Examiner |
|---|---|---|
| Munich | 13 July 2023 | Schmidt, Karsten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 15 7914

This annex lists the patent family members relating to the patent documents cited in the above-mentioned European search report.
The members are as contained in the European Patent Office EDP file on
The European Patent Office is in no way liable for these particulars which are merely given for the purpose of information.

13-07-2023

| Patent document cited in search report | | Publication date | Patent family member(s) | | Publication date |
|---|---|---|---|---|---|
| US 2013054271 | A1 | 28-02-2013 | NONE | | |
| US 2023051982 | A1 | 16-02-2023 | US | 2023051982 A1 | 16-02-2023 |
| | | | WO | 2023019253 A2 | 16-02-2023 |
| CN 105260974 | A | 20-01-2016 | NONE | | |
| US 2007288268 | A1 | 13-12-2007 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MOY, A.J. ; SCHWARTZ, J.M. ; CHEN, R ; SADRI, S. ; LUCAS, E. ; CATO, K.D. ; ROSSETTI, S.C.** Measurement of clinical documentation burden among physicians and nurses using electronic health records: a scoping review. *Journal of the American Medical Informatics Association,* 2021, vol. 28 (5), 998-1008
- The present and future of team documentation: the role of patients, families, and artificial intelligence. **LIN, S.** Mayo Clinic Proceedings. Elsevier, May 2020, vol. 95, 852-855
- **LUTZ, C. ; NEWLANDS, G.** Privacy and smart speakers: A multi-dimensional approach. *The Information Society,* 2021, vol. 37 (3), 147-162
- **BARTH, S. ; IONITA, D. ; HARTEL, P.** Understanding Online Privacy-A Systematic Review of Privacy Visualizations and Privacy by Design Guidelines. *ACM Computing Surveys (CSUR),* 2022, vol. 55 (3), 1-37
- **HUEBNER, A. ; JI, W. ; XIAO, X.** Meeting Summarization with Pre-training and Clustering Methods. *arXiv preprint arXiv:2111.08210,* 2021
- **DELA CRUZ, J.E. ; SHABOSKY, J.C. ; ALBRECHT, M. ; CLARK, T.R. ; MILBRANDT, J.C. ; MARKWELL, S.J. ; KEGG, J.A.** Typed versus voice recognition for data entry in electronic health records: emergency physician time use and interruptions. *Western Journal of Emergency Medicine,* 2014, vol. 15 (4), 541
- **HODGSON, T. ; MAGRABI, F. ; COIERA, E.** Efficiency and safety of speech recognition for documentation in the electronic health record. *Journal of the American Medical Informatics Association,* 2017, vol. 24 (6), 1127-1133
- Medical dialogue summarization for automated reporting in healthcare. **MOLENAAR, S. ; MAAS, L. ; BURRIEL, V. ; DALPIAZ, F. ; BRINKKEMPER, S.** International Conference on Advanced Information Systems Engineering. Springer, Cham, June 2020, 76-88
- **ZHU, H. ; DONG, L. ; WEI, F. ; QIN, B. ; LIU, T.** Transforming wikipedia into augmented data for query-focused summarization. *IEEE/ACM Transactions on Audio, Speech, and Language Processing,* 2022
- Extracting structured data from physician-patient conversations by predicting noteworthy utterances. **KRISHNA, K. ; PAVEL, A. ; SCHLOSS, B. ; BIGHAM, J.P. ; LIPTON, Z.C.** In Explainable AI in Healthcare and Medicine. Springer, Cham, 2021, 155-169
- **LEWIS, M. ; LIU, Y. ; GOYAL, N. ; GHAZVININE-JAD, M. ; MOHAMED, A. ; LEVY, O. ; STOYANOV, V. ; ZETTLEMOYER, L.** Bart: Denoising sequence-to-sequence pre-training for natural language generation, translation, and comprehension. *arXiv preprint arXiv:1910.13461,* 2019
- Medlinker: Medical entity linking with neural representations and dictionary matching. **LOUREIRO, D. ; JORGE, A.M.** European Conference on Information Retrieval. Springer, Cham, April 2020, 230-237
- **ALOMARI, AYHAM et al.** Deep reinforcement and transfer learning for abstractive text summarization: A review. *Computer Speech & Language,* 2022, vol. 71, 101276
- **ZHANG, MENGLI et al.** FAR-ASS: Fact-aware reinforced abstractive sentence summarization. *Information Processing & Management,* 2021, vol. 58 (3), 102478
- **XU, TIANYANG ; CHUNYUN ZHANG.** Reinforced Generative Adversarial Network for Abstractive Text Summarization. *arXiv preprint arXiv:2105.15176,* 2021
- **YANG, YAOSHENG et al.** Distantly supervised NER with partial annotation learning and reinforcement learning. *Proceedings of the 27th International Conference on Computational Linguistics,* 2018
- **LIN, C.Y.** Rouge: A package for automatic evaluation of summaries. *In Text summarization branches out,* July 2004, 74-81
- **SUNIL MOHAN ; RICO ANGELL ; NICHOLAS MONATH ; ANDREW MCCALLUM.** Low Resource Recognition and Linking of Biomedical Concepts from a Large Ontology. *Proceedings of the ACM Conference on Bioinformatics, Computational Biology, and Health Informatics,* 2021
- **OLIVIER BODENREIDER.** The unified medical language system (umls): integrating biomedical terminology. *Nucleic acids research,* 2004, vol. 32, D267-70

- **JIAO LI ; YUEPING SUN ; ROBIN J JOHNSON ; DANIELA SCIAKY ; CHIH-HSUAN WEI ; ROBERT LEAMAN ; ALLAN PETER DAVIS ; CAROLYN J MATTINGLY ; THOMAS C WIEGERS ; ZHIYONG LU.** Biocreative v cdr task corpus: a resource for chemical disease relation extraction. *Database,* 2016
- **NICOLA DE CAO ; GAUTIER IZACARD ; SEBAS-TIAN RIEDEL ; FABIO PETRONI.** Autoregressive entity retrieval. *International Conference on Learning Representations,* 2021
- **REZARTA ISLAMAJ DOGAN ; ROBERT LEAMAN ; ZHIYONG LU.** Ncbi disease corpus: a resource for disease name recognition and concept normalization. *Journal of biomedical informatics,* 2014, vol. 47, 1-10
- COMETA: A corpus for medical entity linking in the social media. **MARCO BASALDELLA ; FANGYU LIU ; EHSAN SHAREGHI ; NIGEL COLLIER.** Pro-ceedings of the 2020 Conference on Empirical Methods in Natural Language Processing (EMNLP). As-sociation for Computational Linguistics, 2020, 3122-3137
- **YUAN, HONGYI ; ZHENG YUAN ; SHENG YU.** Gen-erative Biomedical Entity Linking via Knowledge Base-Guided Pre-training and Synonyms-Aware Fine-tuning. *rXiv preprint arXiv:2204.05164,* 2022
- **YUAN, HONGYI et al.** Biobart: pretraining and evaluation of a biomedical generative language model. *arXiv preprint arXiv:2204.03905,* 2022